(19) Europäisches Patentamt
European Patent Office
Office européen des brevets

(11) **EP 4 491 664 A1**

(12) **EUROPEAN PATENT APPLICATION**
published in accordance with Art. 153(4) EPC

(43) Date of publication:
**15.01.2025 Bulletin 2025/03**

(21) Application number: **23907847.0**

(22) Date of filing: **22.12.2023**

(51) International Patent Classification (IPC):
***C08K 3/30** (2006.01)* ***C08J 3/24** (2006.01)*
***C08F 2/44** (2006.01)* ***C08K 5/17** (2006.01)*
***C08K 5/092** (2006.01)* ***C08K 5/13** (2006.01)*

(52) Cooperative Patent Classification (CPC):
**C08F 2/44; C08J 3/24; C08K 3/30; C08K 5/092; C08K 5/13; C08K 5/17**

(86) International application number:
**PCT/KR2023/021361**

(87) International publication number:
**WO 2024/136564 (27.06.2024 Gazette 2024/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.12.2022 KR 20220183171**
**21.12.2023 KR 20230188595**

(71) Applicant: **LG Chem, Ltd.**
**Yeongdeungpo-gu**
**Seoul 07336 (KR)**

(72) Inventors:
- **LEE, Jin Woo**
  **Daejeon 34122 (KR)**
- **YANG, Seung Hun**
  **Daejeon 34122 (KR)**
- **KIM, Ju Eun**
  **Daejeon 34122 (KR)**
- **CHO, Joonil**
  **Daejeon 34122 (KR)**

(74) Representative: **Plasseraud IP**
**104 Rue de Richelieu**
**CS92104**
**75080 Paris Cedex 02 (FR)**

(54) **SUPERABSORBENT POLYMER AND METHOD FOR PREPARING SAME**

(57) Provided are a super absorbent polymer which has excellent water retention capacity and absorbency under pressure while exhibiting improved bacterial growth inhibition properties and deodorization properties by reducing residual monomers in the super absorbent polymer, and exhibits excellent color properties by suppressing discoloration, and a preparation method thereof.

【FIG. 1】

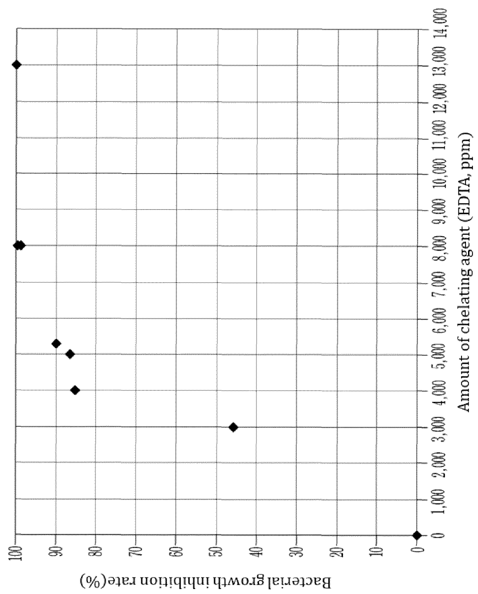


EP 4 491 664 A1

## Description

[TECHNICAL FIELD]

Cross-reference to Related Application(s)

This application claims the benefit of Korean Patent Applications No. 10-2022-0183171 filed on December 23, 2022 and No. 10-2023-0188595 filed on December 21, 2023 with the Korean Intellectual Property Office, the disclosures of which are incorporated herein by reference in their entirety.

The present disclosure relates to a super absorbent polymer and a preparation method thereof, which has excellent water retention capacity and absorbency under pressure while exhibiting improved bacterial growth inhibition properties and deodorization properties by reducing residual monomers in the super absorbent polymer, and exhibits excellent color properties by suppressing discoloration.

[BACKGROUND OF ART]

A super absorbent polymer (SAP) is a type of synthetic polymeric material capable of absorbing 500 to 1000 times its own weight of moisture. Various manufacturers have denominated it with different names, such as SAM (Super Absorbency Material), AGM (Absorbent Gel Material), and the like. Such super absorbent polymers started to be practically applied in sanitary products, and they are now being widely used for water retaining soil products for gardening, water stop materials for the civil engineering and construction, sheets for raising seedling, freshkeeping agents for food distribution fields, materials for poultices, electrical insulation or the like.

Particularly, the super absorbent polymer is being most widely applied for hygienic products or disposable absorbent products such as diapers for children or diapers for adults. Among them, in case applied for diapers for adults, secondary odor due to bacterial growth causes significant discomfort to consumers. In order to solve the problem, previously, an attempt to incorporate various bacterial growth inhibition components, or deodorizing or antibacterial components in the super absorbent polymer has been made.

However, when incorporating antibacterial agent for inhibiting bacterial growth in the super absorbent polymer, it was not easy to select and incorporate antibacterial components which exhibit excellent bacterial growth inhibition property and deodorization property, and yet, are not harmful to the human body, meet economic efficiency, and do not deteriorate basic properties of the super absorbent polymer.

For example, there has been an attempt to incorporate an antibacterial component containing antibacterial metal ion such as silver, copper, and the like, e.g., copper oxide, in the super absorbent polymer. The antibacterial metal ion-containing component may kill bacteria having an enzyme that may destroy cell walls of microorganisms such as bacteria to induce odor in the super absorbent polymer, thereby endowing deodorization property. However, the metal ion-containing component is classified as BIOCIDE substance that may kill even microorganisms beneficial to the human body. Thus, in case the super absorbent polymer is applied to sanitary products such as diapers for children or adults, incorporation of the metal ion-containing antibacterial components is excluded as much as possible.

In addition, previously, when incorporating antibacterial agents for inhibiting bacterial growth in the super absorbent polymer, a method of blending a small amount of the antibacterial agent with the super absorbent polymer was mainly applied. However, in case such a blending method was applied, it was difficult to uniformly maintain the bacterial growth inhibition property as time passes. Moreover, such a blending method may cause non-uniform coatability and delamination of the antibacterial components during the process of blending the super absorbent polymer and the antibacterial agent, and it was required to install new equipment for blending.

Meanwhile, in order for a super absorbent polymer to be used as a sanitary material, the residual monomer (RM) concentration must be low. If RM is high, absorbent performance such as water retention capacity and absorbency under pressure, of the super absorbent polymer cannot be sufficiently exhibited. In order to lower the RM concentration, it is basic to use acrylic acid with a low dimer concentration as the main raw material, and the methods such as increasing the amount of thermal initiator added or adding additional additives that can lower the RM concentration are used. However, there is a problem in that other physical properties of the super absorbent polymer are lowered or the additional addition process is complicated. In particular, when the amount of thermal initiator added is increased, there is a problem in that the super absorbent polymer to be prepared turns yellow.

In addition, a chelating agent is used in the preparation of a super absorbent polymer as a method of improving the deodorizing performance of super absorbent polymer by suppressing the growth of ammonia-producing bacteria. However, when using a chelating agent, there was a problem in that the residual monomer content in the super absorbent polymer to be prepared relatively increased compared to the case where the chelating agent was not used.

Accordingly, there is a continuous demand for the development of technology related to a super absorbent polymer having excellent bacterial growth inhibition properties and deodorizing properties without deteriorating basic

physical properties and absorption performance by lowering the residual monomer content in the super absorbent polymer.

[DETAILED DESCRIPTION OF THE INVENTION]

[Technical Problem]

The present disclosure relates to a super absorbent polymer and a preparation method thereof, which has excellent water retention capacity and absorbency under pressure while exhibiting improved bacterial growth inhibition properties and deodorization properties by reducing residual monomers in the super absorbent polymer, and exhibits excellent color properties by suppressing discoloration.

The present disclosure relates to a product, specifically a sanitary product, which has excellent water retention capacity and absorbency under pressure while exhibiting improved bacterial growth inhibition properties and deodorizing properties by including the above-described super absorbent polymer.

[Technical Solution]

In order to solve the above problems, there is provided a super absorbent polymer including

a base resin powder including a first cross-linked polymer of a water-soluble ethylene-based unsaturated monomer having at least partially neutralized acidic groups, and a chelating agent; and

a surface cross-linked layer formed on the base resin powder and including a second cross-linked polymer in which the first cross-linked polymer is further cross-linked using a surface cross-linking agent,

wherein the following conditions (i) to (iii) are satisfied:

(i) CFU(12h), which is the number of individuals of proliferated bacteria per unit volume of synthetic urine (CFU/ml) obtained by adding 2 g of the super absorbent polymer to 50 ml of synthetic urine inoculated with 3000 CFU/ml of bacteria of Proteus mirabilis (ATCC29906), and then incubating at 35 °C for 12 hours: $10^9$ or less

(ii) Residual monomer content measured according to the EDANA WSP 210.3: 450 ppm or less

(iii) Hunter color B: 10 or less.

In addition, there is also provided a preparation method of the super absorbent polymer, including the steps of forming a hydrogel polymer by performing cross-linking polymerization of a water-soluble ethylene-based unsaturated monomer having at least partially neutralized acidic groups in the presence of an internal cross-linking agent and a persulfate, followed by chopping; forming a base resin powder by drying and pulverizing the hydrogel polymer; and cross-linking a surface of the base resin powder by heat treating the base resin powder in the presence of a surface cross-linking agent,

wherein the persulfate is added in an amount of 1,800 to 3,500 ppm based on a total weight of the water-soluble ethylene-based unsaturated monomer, and

a chelating agent is additionally added in an amount of 6,000 to 15,000 ppm based on a total weight of the water-soluble ethylene-based unsaturated monomer in at least one process of the cross-linking polymerization and chopping.

Further, there is provided a product, specifically a sanitary product, including the above-described super absorbent polymer.

[ADVANTAGEOUS EFFECTS]

The super absorbent polymer according to the present disclosure can exhibit particularly excellent antibacterial and deodorizing properties against bacteria that are harmful to the human body and cause secondary odor, such as Proteus mirabilis. Accordingly, the super absorbent polymer may be very preferably applied to various sanitary products such as diapers for adults, where secondary odor is a particular problem.

Further, according to the preparation method of a super absorbent polymer of the present disclosure, there is provided a super absorbent polymer, which has excellent water retention capacity and absorbency under pressure while exhibiting improved bacterial growth inhibition properties and deodorization properties by reducing residual monomers in the super absorbent polymer, and exhibits excellent color properties by suppressing discoloration.

[BRIEF DESCRIPTION OF THE DRAWINGS]

FIG. 1 is a graph showing the results of measuring the bacterial growth inhibition rate according to the amount of chelating agent (EDTA-Na) added in Experimental Example 2.

FIG. 2 is a graph showing the results of observing the change in the amount of chelating agent detected in the super absorbent polymer according to the amount of chelating agent added during the preparation of the super absorbent polymer through HPLC analysis.

[DETAILED DESCRIPTION OF THE EMBODIMENTS]

The terminology used herein is for the purpose of describing particular embodiments only and is not intended to limit the invention.

The singular forms are intended to include the plural forms as well, unless the context clearly indicates otherwise. It will be further understood that the terms "include", "have", or "possess" when used in this specification, specify the presence of stated features, steps, components, or combinations thereof, but do not preclude the presence or addition of one or more other features, steps, components, or combinations thereof.

The terms "the first", "the second", "the third", and the like are used to describe a variety of components, and these terms are merely employed to distinguish a certain component from other components.

As the present invention can be variously modified and have various forms, specific embodiments thereof are shown by way of examples and will be described in detail. However, it is not intended to limit the present invention to the particular form disclosed and it should be understood that the present invention includes all modifications, equivalents, and replacements within the idea and technical scope of the present invention.

The terminology "polymer" in the present disclosure is in a state in which a water-soluble ethylene-based unsaturated monomer is polymerized, and may include all moisture content ranges, or all particle diameter ranges. Among the polymers, a polymer having a moisture content of about 40 wt% or more after polymerization and before drying may be referred to as a hydrogel polymer, and particles in which the hydrogel polymer is pulverized and dried may be referred to as a cross-linked polymer.

In addition, the terminology "super absorbent polymer powder" refers to a particulate material including a cross-linked polymer in which a water-soluble ethylene-based unsaturated monomer is polymerized and cross-linked by an internal cross-linking agent.

In addition, the terminology "super absorbent polymer" is used to encompass all of a cross-linked polymer in which a water-soluble ethylene-based unsaturated monomer is polymerized or a base resin in the form of powder consisting of super absorbent polymer particles in which the cross-linked polymer is pulverized, and the cross-linked polymer or the base resin further processed, for example, surface cross-linking, fine reassembling, drying, pulverization, classification, etc., to be in a state suitable for commercialization, depending on the context.

Hereinafter, the super absorbent polymer and the preparation method thereof will be described in more detail according to specific embodiments of the present invention.

**Super absorbent polymer**

The super absorbent polymer according to the present disclosure includes

a base resin powder including a first cross-linked polymer of a water-soluble ethylene-based unsaturated monomer having at least partially neutralized acidic groups, and a chelating agent; and

a surface cross-linked layer formed on the base resin powder and including a second cross-linked polymer in which the first cross-linked polymer is further cross-linked using a surface cross-linking agent,

wherein the following conditions (i) to (iii) are satisfied:

(i) CFU(12h), which is the number of individuals of proliferated bacteria per unit volume of synthetic urine (CFU/ml) obtained by adding 2 g of the super absorbent polymer to 50 ml of synthetic urine inoculated with 3000 CFU/ml of bacteria of Proteus mirabilis (ATCC29906), and then incubating at 35 °C for 12 hours: $10^9$ or less

(ii) Residual monomer content measured according to the EDANA WSP 210.3: 450 ppm or less

(iii) Hunter color B: 10 or less.

The super absorbent polymer may have CFU(12h) of $1 \times 10^9$ or less, more specifically, $9 \times 10^8$ or less, or $8.5 \times 10^8$ or less, wherein the CFU(12h) is the number of individuals of proliferated bacteria per unit volume of synthetic urine

(CFU/ml) obtained by adding 2 g of the super absorbent polymer to 50 ml of synthetic urine inoculated with 3000 CFU/ml of bacteria of Proteus mirabilis (ATCC29906), and then incubating at 35 °C for 12 hours. The smaller the number of proliferated bacteria, the better the antibacterial properties, so the lower limit is not specified, but may be $1 \times 10^3$ or more, or $1 \times 10^6$ or more.

Meanwhile, a composition of synthetic urine used when evaluating the antibacterial properties of the super absorbent polymer does not directly affect the antibacterial properties of the super absorbent polymer according to the present disclosure. Accordingly, in the present disclosure, it can be used as the synthetic urine without particular limitations, as long as it includes NaCl, urea, and polyvalent metals.

For example, when evaluating antibacterial properties in the present disclosure, synthetic urine including 0.1 to 1.5 wt%, more specifically 0.5 to 1 wt% of NaCl, 0.5 to 5 wt%, more specifically 1 to 3 wt% of urea, and 0.01 to 0.5 wt%, more specifically 0.05 to 0.3 wt% of a polyvalent metal based on the total weight of synthetic urine can be used. At this time, the content of each component was calculated from the amount of raw materials of NaCl, urea, and polyvalent metal used in the production of synthetic urine.

Meanwhile, the polyvalent metal may specifically be one or more divalent metals such as calcium and magnesium. Accordingly, the raw material of the polyvalent metal may be a salt (carbonate, chloride, etc.) including the above-mentioned polyvalent metal or a hydrate thereof. The composition and preparation method of the synthetic urine used in the present disclosure are as described in Experimental Examples below.

In addition, the super absorbent polymer according to the present disclosure has an RM (residual monomer) content measured according to the EDANA WSP 210.3 of 500 ppm or less, 400 ppm or less, or 380 ppm or less based on the total weight of the super absorbent polymer. As the lower RM content can be evaluated as the better, there is no specific lower limit. However, the RM content may be 100 ppm or more.

The RM content in the super absorbent polymer can be measured through HPLC analysis, and specific measurement method and conditions are as described in Experimental Examples below.

In addition, the super absorbent polymer according to the present disclosure exhibits excellent discoloration resistance, and the resulting Hunter color B may be 10 or less, 9 or less, or 8.7 or less, and 5 or more, 6 or more, or 7 or more.

The Hunter color B of the super absorbent polymer may be measured using a colorimeter, and specific measurement method and conditions are as described in Experimental Examples below.

In addition, the super absorbent polymer has a bacterial growth inhibition rate represented by the following Equation 1 of 95% or more, more specifically, 99% or more, or 99.4% or more:

[Equation 1]

$$\text{Bacterial growth inhibition rate} = [1- \{CFU(12h)/ \text{ CFU control}(12h)\}]*100\ (\%)$$

in Equation 1, CFU(12 h) represents the number of individuals of proliferated bacteria per unit volume of synthetic urine (CFU/ml), which was obtained by adding 2 g of the super absorbent polymer to 50 ml of synthetic urine inoculated with 3000 CFU/ml of bacteria of Proteus mirabilis (ATCC 29906), and then incubating at 35 °C for 12 hours, and

CFU control(12 h) represents the number of individuals of proliferated bacteria per unit volume of synthetic urine (CFU/ml), which was obtained by incubating synthetic urine inoculated with the above bacteria on the super absorbent polymer of a control group under the same conditions, wherein the super absorbent polymer of the control group refers to the same super absorbent polymer except that the base resin powder does not include a chelate.

In addition, the super absorbent polymer exhibits excellent bacterial growth inhibition rate, reduced residual monomer content, and color characteristics as described above, as well as excellent absorption performance. Specifically, the super absorbent polymer may further satisfy the following conditions (iv) to (vi):

(iv) Centrifuge retention capacity (CRC) measured according to the EDANA WSP 241.2: 39 g/g or more
(v) Absorbency under pressure (AUP) at 4826 Pa (0.7 psi) measured according to the EDANA WSP 242.3-10: 17 g/g or more
(vi) Arithmetic average of centrifuge retention capacity and absorbency under pressure (EFFC) represented by the following Equation 2: 28 or more

[Equation 2]

$$EFFC = (CRC + AUP)/2$$

in Equation 2, CRC is centrifuge retention capacity of the super absorbent polymer to saline (0.9 wt% aqueous solution of sodium chloride) for 30 minutes measured according to the EDANA WSP 241.2, and

AUP is absorbency under pressure of the super absorbent polymer to saline (0.9 wt% aqueous solution of sodium chloride) at 4826 Pa (0.7 psi) for 1 hour measured according to the EDANA WSP 242.3-10.

Specifically, the super absorbent polymer may have CRC to saline (0.9 wt% aqueous solution of sodium chloride) for 30 minutes measured according to the EDANA WSP 241.3 of 39 g/g or more, or 40g/g or more, and 50g/g or less, or 45 g/g or less.

Further, the super absorbent polymer may have absorbency under pressure (AUP) to saline (0.9 wt% aqueous solution of sodium chloride) at 4826 Pa (0.7 psi) measured according to the EDANA WSP 242.3-10 of 17 g/g or more, 21 g/g or more, or 21.2 g/g or more, and 25g/g or less, or 23g/g or less.

Furthermore, the super absorbent polymer may have EFFC defined by the Equation 3 of 28 or more, and 32 or less, or 31.8 or less.

As CRC and AUP, which are in a trade-off relationship, satisfy the above optimal range conditions, and EFFC, which is the arithmetic average of CRC and AUP, satisfies the above range condition, the super absorbent polymer prepared by the preparation method of the present disclosure exhibits excellent absorption performance and is suitable for application to sanitary products.

Meanwhile, specific measurement methods and conditions of CRC and AUP are as described in Experimental Examples below.

As the super absorbent polymer exhibits an excellent growth inhibition effect against Proteus mirabilis, which generates secondary odor through ammonia production, it can also have an excellent deodorizing effect against odors, specifically ammonia, caused by Proteus mirabilis among various bacteria present on the skin.

In addition, the super absorbent polymer prepared by the preparation method according to the present disclosure includes a base resin powder including a first cross-linked polymer of a water-soluble ethylene-based unsaturated monomer having at least partially neutralized acidic groups, and a chelating agent; and a surface cross-linked layer formed on the base resin powder and including a second cross-linked polymer in which the first cross-linked polymer is further cross-linked using a surface cross-linking agent.

The chelating agent may exist dispersed in the base resin powder or on the surface of the base resin powder. More specifically, the chelating agent may be dispersed in the first cross-linked polymer in the base resin powder, or may be dispersed on the surface of the base resin powder. As the chelating agent is uniformly dispersed and included in the super absorbent polymer, improved bacterial growth inhibition properties and deodorizing properties can be consistently and safely exhibited without deteriorating physical properties of the super absorbent polymer, such as water retention capacity and absorbency under pressure.

The chelating agent may be included in an amount of 4,500 to 12,000 ppm based on the total weight of the super absorbent polymer.

When included within the above range, the super absorbent polymer exhibits sufficient antibacterial properties, and the condition (i) of the super absorbent polymer can be easily achieved. More specifically, the chelating agent may be used in an amount of 4,500 ppm or more, 5,000 ppm or more, 5,500 ppm or more, or 6,000 ppm or more, and 12,000 ppm or less, 11,000 ppm or less, or 10,000 ppm or less based on the total weight of the super absorbent polymer.

The chelating agent content in the super absorbent polymer can be measured by HPLC analysis, and specific measurement method is as described in Experimental Examples below.

The type of the chelating agent will be described in detail in the preparation method below.

Further, in the super absorbent polymer according to the present disclosure, the base resin powder may further include a persulfate used as a thermal polymerization initiator during production.

The type and input of the persulfate are described in detail in the preparation method below.

The super absorbent polymer according to the present disclosure can exhibit particularly excellent antibacterial and deodorizing properties against bacteria that are harmful to the human body and cause secondary odor, such as Proteus mirabilis. Accordingly, the super absorbent polymer can be preferably included and used in various sanitary products, such as diapers for children, diapers for adults, or sanitary pads. In particular, it may be very preferably applied to diapers for adults, where secondary odor caused by bacterial growth is a particular problem.

**Preparation method of super absorbent polymer**

The preparation method of the super absorbent polymer according to the present disclosure includes the steps of:

forming a hydrogel polymer by performing cross-linking polymerization of a water-soluble ethylene-based unsaturated monomer having at least partially neutralized acidic groups in the presence of an internal cross-linking agent and a persulfate, followed by chopping (step 1);
forming a base resin powder by drying and pulverizing the hydrogel polymer (stetp 2); and
cross-linking a surface of the base resin powder by heat treating the base resin powder in the presence of a surface

cross-linking agent (step 3),

wherein the persulfate is added in an amount of 1,800 to 3,500 ppm based on a total weight of the water-soluble ethylene-based unsaturated monomer, and

a chelating agent is additionally added in an amount of 6,000 to 15,000 ppm based on a total weight of the water-soluble ethylene-based unsaturated monomer in at least one process of the cross-linking polymerization and chopping.

When a chelating agent is introduced to impart deodorizing properties to a super absorbent polymer, the growth of ammonia-producing bacteria that cause bad odors can be suppressed, but there is a problem in that the residual monomer (RM) content in the super absorbent polymer increases. When the RM content in the super absorbent polymer is high, basic physical properties and absorption performance of the super absorbent polymer are deteriorated, making it unusable as a sanitary material. Accordingly, a method of increasing the amount of thermal initiator added is used to lower the RM content, but an excessive amount of thermal initiator reduces discoloration resistance of the super absorbent polymer, causing the super absorbent polymer to turn yellow and its physical properties to also deteriorate.

Accordingly, in the present disclosure, the preparation of a super absorbent polymer exhibiting antibacterial and deodorizing properties using a chelating agent uses a persulfate (SPS), which has an excellent thermal initiator effect, and simultaneously controls the input of the persulfate and the chelating agent. Accordingly, the residual monomer content in the super absorbent polymer is lowered, so that it is possible to produce a super absorbent polymer that exhibits excellent water retention capacity and absorbency under pressure while having improved bacterial growth inhibition properties and deodorization properties, and also suppresses discoloration thereby exhibiting excellent color.

Hereinafter, the preparation method according to the present invention will be described in detail step by step.

**Step 1**

In the preparation method according to the present invention, the step 1 is a step of forming a hydrogel polymer.

Specifically, the hydrogel polymer may be prepared by performing cross-linking polymerization of a water-soluble ethylene-based unsaturated monomer having at least partially neutralized acidic groups in the presence of an internal cross-linking agent and a persulfate, and chopping the resulting product, wherein a chelating agent is added in an optimal amount during the cross-linking polymerization or chopping.

When a chelating agent is added during cross-linking polymerization, the chelating agent is uniformly dispersed in the cross-linked polymer (hereinafter, referred to as 'first cross-linked polymer'), and as a result, antibacterial properties and deodorizing properties due to inhibition of bacterial growth may be exhibited uniformly for a long time. In addition, the chelating agent can improve gel strength by combining with metal ions in the cross-linking polymerization and prevent the polymer from being oxidized by metal ions in the cross-linking structure.

Meanwhile, when a chelating agent is added during chopping, the chelating agent may be added as an aqueous solution dissolved in water. Accordingly, the chelating agent is dispersed on the surface of the base resin powder, and as a result, it can be uniformly distributed in the final super absorbent polymer. Accordingly, antibacterial properties and deodorizing properties due to inhibition of bacterial growth can be uniformly exhibited for a long time.

Specifically, the chelating agent may be one or more selected from the group consisting of ethylenediamine tetraacetic acid (EDTA), diethylenetriamine pentaacetic acid (DTPA), phenolic acid, citric acid, amino acid, and a salt thereof. More specifically, ethylenediamine tetraacetic acid (EDTA), diethylenetriamine pentaacetic acid (DTPA), or a sodium salt thereof may be used, and more specifically, a tetravalent sodium salt of ethylenediamine tetraacetic acid (EDTA) or diethylenetriamine pentaacetic acid (DTPA) may be used.

Meanwhile, unlike conventional antibacterial agents such as Ag, Zn, Cu, or a tetravalent ammonium salt thereof that kill bacteria by destroying cell walls, the chelating agent used in the present disclosure only inhibits bacterial growth by chelating multivalent metals (Ca, Mg ions) necessary for bacterial growth. Accordingly, cell deformation and death of beneficial bacteria that may occur when bacteria are killed can be prevented.

In particular, the chelating agent used in the present disclosure can exhibit an excellent growth inhibition effect against Gram-negative bacteria, especially Proteus mirabilis, which generates secondary odor through ammonia production. As a result, the super absorbent polymer to be prepared may exhibit an excellent antibacterial effect against Proteus mirabilis, as well as a deodorizing effect against odor, specifically ammonia, caused by Proteus mirabilis.

The chelating agent is added in an amount of 6,000 to 15,000 ppm based on the total weight of the water-soluble ethylene-based unsaturated monomer.

When the amount of the chelating agent is less than 6,000 ppm, it is difficult to obtain sufficient antibacterial and deodorizing properties. When the amount of the chelating agent exceeds 15,000 ppm, the RM content in the super absorbent polymer increases due to the excess chelating agent, and as a result, physical properties and absorption performance of the super absorbent polymer are deteriorated. More specifically, the amount of the chelating agent may be 6,000ppm or more, 7,000ppm or more, 7,500ppm or more, or 8,000ppm or more, and 15,000ppm or less, 14,000ppm or

less, 13,000ppm or less, 10,000 ppm or less, or 9,000 ppm or less based on the total weight of the water-soluble ethylene-based unsaturated monomer.

In addition, considering the physical properties and absorption performance of the super absorbent polymer to be finally prepared, and the function of the chelating agent and persulfate on antibacterial and deodorizing performance, the above effect can be further enhanced by controlling the relative mixing ratio as long as each content range is satisfied. Specifically, in the preparation method of a super absorbent polymer according to the present disclosure, when a chelating agent is additionally added during cross-linking polymerization, the weight ratio of the persulfate and the chelating agent may preferably be 1:3 to 1:4, or 1:3.5 to 1:4.

In addition, when a chelating agent is additionally added during chopping, the weight ratio of the persulfate and the chelating agent may preferably be 1:2 to 1:7, or 1:2.5 to 1:6.5.

In addition, when a chelating agent is added in both the cross-linking polymerization and chopping processes, the weight ratio of the persulfate and the chelating agent may preferably be 1:2 to 1:5, or 1:3 to 1:4.

Hereinafter, the formation of a hydrogel polymer in step 1 will be described in detail.

When preparing the hydrogel polymer, a monomer composition in a solution state including a water-soluble ethylene-based unsaturated monomer having at least partially neutralized acidic groups, a persulfate, and a solvent may be used.

Additionally, when a chelating agent is added during cross-linking polymerization, it may be added during the production of the monomer composition. In this case, the monomer composition may include a water-soluble ethylene-based unsaturated monomer having at least partially neutralized acidic groups, a persulfate, a solvent, and a chelating agent. The type and input of the chelating agent are the same as described above.

The water-soluble ethylene-based unsaturated monomer may be any monomer commonly used in the preparation of a super absorbent polymer. For example, the water-soluble ethylene-based unsaturated monomer may be a compound represented by the following Chemical Formula 1:

[Chemical Formula1] R-COOM'

in Chemical Formula 1,

R is a C2 to C5 alkyl group having an unsaturated bond, and
M' is a hydrogen atom, a monovalent or divalent metal, an ammonium group, or an organic amine salt.

Preferably, the monomer may be at least one selected from the group consisting of acrylic acid, methacrylic acid, and a monovalent metal salt, a divalent metal salt, an ammonium salt and an organic amine salt of the acid. When (meth) acrylic acid and/or a salt thereof is used as a water-soluble ethylene-based unsaturated monomer, it is advantageous to obtain a super absorbent polymer having improved absorption performance. In addition, maleic anhydride, fumalic acid, crotonic acid, itaconic acid, 2-acryloylethane sulfonic acid, 2-methacryloylethane sulfonic acid, 2-(meth)acryloylpropanesulfonic acid, 2-(meth)acrylamide-2-methyl propanesulfonic acid, (meth)acrylamide, N-substituted (meth)acrylate, 2-hydroxyethyl(meth)acrylate, 2-hydroxypropyl(meth)acrylate, methoxypolyethyleneglycol(meth)acrylate, polyethyleneglycol(meth)acrylate, (N,N)-dimethylaminoethyl(meth)acrylate, (N,N)-dimethylaminopropyl(meth)acrylamide and the like may be used as the monomer.

The water-soluble ethylene-based unsaturated monomer has acidic groups, and at least some of the acidic groups may be neutralized by a neutralizing agent.

In the preparation method according to the present disclosure, neutralization of at least some of the acidic groups of the water-soluble ethylene-based unsaturated monomer may be performed by adding a neutralizing agent during the process of preparing a monomer composition in which the water-soluble ethylene-based unsaturated monomer having the acidic groups, an internal cross-linking agent, sodium persulfate, and optionally a chelating agent are mixed. The monomer composition prepared therefrom includes a water-soluble ethylene-based unsaturated monomer having at least partially neutralized acidic groups, an internal cross-linking agent, and sodium persulfate, and may optionally further include a chelating agent.

During the neutralization, it is preferable to appropriately determine the concentration of the water-soluble ethylene-based unsaturated monomer having acidic groups in consideration of the polymerization time and reaction conditions in the subsequent polymerization reaction. For example, the concentration of the water-soluble ethylene-based unsaturated monomer in the mixture including the water-soluble ethylene-based unsaturated monomer having acidic groups, an internal cross-linking agent, a polymerization initiator, and a neutralizing agent may preferably be 20 to 60 wt%, specifically 20 wt% or more, and 60 wt% or less, or 40 wt% or less.

In addition, the neutralizing agent may be at least one basic substance such as sodium hydroxide, potassium hydroxide, ammonium hydroxide, etc., which can neutralize acidic groups.

The degree of neutralization of the water-soluble ethylene-based unsaturated monomer refers to the degree of

neutralization of the acidic groups included in the water-soluble ethylene-based unsaturated monomer by the neutralizing agent. An excessively high degree of neutralization causes the neutralized monomers to be precipitated, and thus polymerization may not readily occur. On the contrary, an excessively low degree of neutralization not only deteriorates absorbency of the polymer, but also gives the polymer hard-to-handle properties, such as those of an elastic rubber. Accordingly, it is desirable to appropriately select the degree of neutralization of the water-soluble ethylene-based unsaturated monomer according to the physical properties of the super absorbent polymer desired in this disclosure. For example, the degree of neutralization of the water-soluble ethylene-based unsaturated monomer may be 50 to 90 mol%, more specifically, 50 mol% or more, 60 mol% or more, or 65 mol% or more, and 90 mol% or less, 85 mol% or less, 80 mol% or less, or 75 mol% or less.

Meanwhile, the terminology 'internal cross-linking agent' used herein is different from "a surface cross-linking agent" for cross-linking the surface of the base resin to be described later, and the internal cross-linking agent, and serves to form a polymer including a cross-linked structure by introducing cross-linking bonds between the unsaturated bonds of the water-soluble ethylene-based unsaturated monomers described above.

The cross-linking in the above cross-linking polymerization step proceeds on the surface and inside, but when the surface cross-linking process of the base resin to be described later is in progress, the surface of the particles of the finally prepared super absorbent polymer has a structure further cross-linked by a surface cross-linking agent, and the inside of the particles has a structure cross-linked by the internal cross-linking agent.

The internal cross-linking agent may be a cross-linking agent having one or more ethylene-based unsaturated groups in addition to at least one functional group which may react with the water-soluble substituents of the water-soluble ethylene-based unsaturated monomer; or a cross-linking agent having two or more functional groups which may react with the water-soluble substituents of the monomer and/or the water-soluble substituents formed by hydrolysis of the monomer.

As the specific example of the internal cross-linking agent, a C8-C12 bisacrylamide, bismethacrylamide, a poly(meth)acrylate of C2-C10 polyol, a poly(meth)allylether of C2-C10 polyol, or the like may be used. More specifically, at least one selected from the group consisting of N,N'-methylenebis(meth)acrylate, ethyleneoxy(meth)acrylate, polyethyleneoxy(meth)acrylate, polyethylene glycol diacrylate (PEGDA), polypropyleneoxy(meth)acrylate, glycerin diacrylate, glycerin triacrylate, trimethylol triacrylate, triallylamine, triallyl cyanurate, triallyl isocyanate, polyethylene glycol, diethylene glycol, and propylene glycol may be used.

The internal cross-linking agent may be used in an amount of 1000 to 5000 ppm based on the total weight of the water-soluble ethylene-based unsaturated monomer.

When the content of the internal cross-linking agent is too low, cross-linking does not occur sufficiently, and thus it may be difficult to achieve strength above an appropriate level, and when the content of the internal cross-linking agent is too high, the internal cross-linking density increases, and thus it may be difficult to achieve a desired level of water retention capacity. More specifically, the internal cross-linking agent may be used in an amount of 1000 ppm or more, 1200 ppm or more, or 1400 ppm or more, and 5000 ppm or less, 3000 ppm or less, or 2000 ppm or less based on the total weight of the water-soluble ethylene-based unsaturated monomer.

In addition, the persulfate may include sodium persulfate or potassium persulfate, and any one or a mixture of two or more of these may be used.

Among them, sodium persulfate ($Na_2S_2O_8$) promotes the polymerization reaction and allows additional polymerization reaction to proceed during drying after completion of UV polymerization, thereby preventing an increase in the RM content in the super absorbent polymer due to the use of the chelating agent

Meanwhile, if the amount of persulfate added is too high, it may cause discoloration of the super absorbent polymer, so it is necessary to control the amount of persulfate in consideration of the amount of chelating agent added.

Specifically, the persulfate may be added in an amount of 1,800 to 3,500 ppm based on the total weight of water-soluble ethylene-based unsaturated monomer. When the amount of persulfate added is less than 1,800 ppm, it is difficult to sufficiently suppress the increase in RM due to the addition of the chelating agent. When the amount of persulfate added exceeds 3,500 ppm, discoloration resistance of the super absorbent polymer is reduced due to the excessive amount of persulfate, thereby increasing the Hunter color B value. More specifically, the persulfate may be added in an amount of 1,800 ppm or more, 1,900 ppm or more, or 2,000 ppm or more, and 3,000 ppm or less, or 2,800 ppm or less based on the total weight of the water-soluble ethylene-based unsaturated monomer.

Additionally, when preparing the hydrogel polymer, a polymerization initiator may be further used. That is, the polymerization initiator may be further included in the monomer composition.

The persulfate used to prepare the hydrogel polymer of the present disclosure can act as a thermal polymerization initiator, but an additional polymerization initiator can be used to promote the polymerization reaction.

The polymerization initiator may be an initiator for thermal polymerization or an initiator for photopolymerization according to the polymerization method. However, even when the photopolymerization method is applied thereto, a certain amount of heat is generated by UV radiation and the like, and some heat occurs as the polymerization reaction, an exothermal reaction, progresses. Therefore, a thermal polymerization initiator can be further included.

As the thermal polymerization initiator, known compounds other than persulfate can be used. Specifically, one or more compounds selected from the group consisting of an azo-based initiator, hydrogen peroxide, and ascorbic acid may be used. Specifically, 2,2-azobis(2-amidinopropane) dihydrochloride, 2,2-azobis-(N,N-dimethylene)isobutyramidine dihydrochloride, 2-(carbamoylazo)isobutylonitril, 2,2-azobis[2-(2-imidazolin-2-yl)propane] dihydrochloride, 4,4-azobis-(4-cyanovaleric acid), and the like may be used as examples of azo-based initiators. More various thermal polymerization initiators are well disclosed in "Principle of Polymerization (Wiley, 1981)" written by Odian, p 203, and the present disclosure is not limited thereto.

For example, the photopolymerization initiator may be one or more compounds selected from the group consisting of benzoin ether, dialkyl acetophenone, hydroxyl alkylketone, phenyl glyoxylate, benzyl dimethyl ketal, acyl phosphine, and $\alpha$-aminoketone. Further, as the specific example of acyl phosphine, diphenyl(2,4,6-trimethylbenzoyl) phosphine oxide, bis(2,4,6-trimethylbenzoyl) phenylphosphine oxide, ethyl(2,4,6-trimethylbenzoyl) phenylphosphinate, and the like may be used. More various photopolymerization initiators are well disclosed in "UV Coatings: Basics, Recent Developments and New Application (Elsevier, 2007)" written by Reinhold Schwalm, p 115, and the present disclosure is not limited thereto.

In general, when preparing a super absorbent polymer, if the concentration of the polymerization initiator is excessively low, the polymerization rate may become slow, and a large amount of RM may be extracted into the final super absorbent polymer. Conversely, if the concentration of the polymerization initiator is too high, the polymer chains forming the network become shorter, the content of water-soluble components increases, and physical properties such as absorbency under pressure of the polymer may decrease. In addition, considering that SPS is used in an amount of 1,800 to 3,500 ppm based on the total weight of the water-soluble ethylene-based unsaturated monomer when preparing the hydrogel polymer in the present disclosure, when the polymerization initiator is further used, it is preferable that the polymerization initiator is used in an amount of 100 to 5,000 ppm based on the total weight of the water-soluble ethylene-based unsaturated monomer. Meanwhile, when the thermal polymerization initiator and the photopolymerization initiator are used simultaneously, the above content refers to the total mixed content.

Additionally, in the preparation method of the present disclosure, additives such as surfactants, thickeners, plasticizers, stabilizers, and antioxidants may be further used when preparing the hydrogel polymer. That is, the above additives may be further included in the monomer composition.

As the surfactant, it is preferable to use an anionic surfactant. Specifically, the surfactant may include an $SO_3^-$ anion, and a compound represented by the following Chemical Formula 2 may be used.

[Chemical Formula 2] R-SOsNa

In Chemical Formula 2,

R is C8 to 16 alkyl.

In addition, the surfactant may be used in an amount of 300 ppm or less, more specifically, 100 ppm or more, or 150 ppm or more, and 300 ppm or less based on the total weight of the water-soluble ethylene-based unsaturated monomer.

Further, polycarboxylic acid salt may be used as the stabilizer. Specifically, 2-Propenoic acid, 2-methyl, polymer with alpha-(2-methyl-1-oxo-2-propenyl-omega-methoxypoly(oxy-1,2-ethanediyl and 22-Propenoic acid (cas no. 381686-36-8), etc. can be used, and a corresponding commercial product such as ACYMA-GK (manufactured by SAN NOPCO KOREA LTD) can also be used. The stabilizer may be used in an amount of 1500 ppm or less, more specifically, 100 ppm or more, 300 ppm or more, or 500 ppm or more, and 1500 ppm or less, 1300 ppm or less, 1000 ppm or less, or 900 ppm or less based on the total weight of the water-soluble ethylene-based unsaturated monomer.

This monomer composition may be prepared in the form of a solution in which the above-described monomers, internal cross-linking agent, chelating agent, sodium persulfate, and other additives are dissolved in a solvent.

At this time, any solvent which can dissolve the above components may be used without limitation, and for example, water, ethanol, ethyleneglycol, diethyleneglycol, triethyleneglycol, 1,4-butanediol, propyleneglycol, ethyleneglycol monobutylether, propyleneglycol monomethylether, propyleneglycol monomethylether acetate, methylethylketone, acetone, methylamylketone, cyclohexanone, cyclopentanone, diethyleneglycol monomethylether, diethyleneglycol ethylether, toluene, xylene, butyrolactone, carbitol, methylcellosolve acetate, N,N-dimethylacetamide, or a mixture thereof can be used.

The solvent may be included in the monomer composition at a residual quantity except for the above components.

The formation of the hydrogel polymer by polymerizing the monomer composition may be performed by a conventional polymerization method, and the process is not particularly limited. As a non-limiting example, the polymerization method is largely divided into the thermal polymerization and the photopolymerization according to an energy source of the polymerization. In the case of thermal polymerization, it is generally carried out in a reactor equipped with an agitation spindle, such as a kneader. In the case of photopolymerization, it may be carried out in a reactor equipped with a

movable conveyor belt.

For example, the hydrogel polymer may be obtained by introducing the monomer composition into a reactor equipped with an agitation spindle such as a kneader, followed by supplying hot air, or heating the reactor to perform thermal polymerization. The hydrogel polymer may be discharged to a reactor outlet in the form of particles with several centimeters to several millimeters depending on a shape of the agitation spindle provided in the reactor. Specifically, a shape of the hydrogel polymer obtained may vary depending on the concentration and injection rate of the monomer mixture to be injected, and a hydrogel polymer having a weight average particle diameter of 2 to 50 mm may be usually obtained.

As another example, when photopolymerization is performed on the monomer composition in the reactor equipped with a movable conveyor belt, a hydrogel polymer in the form of a sheet may be obtained. At this time, a thickness of the sheet may vary depending on the concentration and injection rate of the monomer composition to be injected. It is preferable to adjust the thickness to 0.5 to 5 cm in order to ensure the production rate while allowing the entire sheet to be polymerized evenly.

After completion of the cross-linking polymerization, a chopping process is performed on the resulting polymerization product.

The chopping process is distinguished from the pulverizing process performed after the subsequent drying process in that the cross-linked polymer obtained as a result of cross-linking polymerization is chopped in a hydrated state before drying.

Additionally, in the present disclosure, a chelating agent may be further added when chopping the cross-linked polymer. At this time, the type, method, and input of the chelating agent added are the same as described above.

The chopping may be performed using a conventional chopping method using a chopping device, except that a chelating agent is added. In the present disclosure, a chopper is preferred because it facilitates the introduction of the chelating agent and enables homogeneous mixing of the cross-linked polymer and the chelating agent.

The hydrogel polymer formed in this way may exhibit a moisture content of 40 to 80 wt%. At this time, the moisture content is the weight occupied by moisture in the total weight of the hydrogel polymer, and it means a value of which the weight of the dried polymer is subtracted from the weight of the hydrogel polymer. Specifically, the moisture content is defined as a value calculated by measuring the weight loss due to moisture evaporation from the polymer in the process of increasing the temperature of the polymer for drying through infrared heating. At this time, the drying conditions are maintained at room temperature at about 180 °C for about 40 minutes to measure the moisture content.

In addition, in the preparation method according to the present disclosure, when a chelating agent is added during the cross-linking polymerization process, the hydrogel polymer to be prepared is a cross-linked polymer in which a water-soluble ethylene-based unsaturated monomer having at least partially neutralized acidic groups is cross-linking polymerized in the presence of an internal cross-linking agent, a persulfate, and optionally a chelating agent. Accordingly, the hydrogel polymer has a three-dimensional network structure in which the main chains formed by polymerizing the monomers are cross-linked by the internal cross-linking agent, and the chelating agent is uniformly dispersed and distributed inside and on the surface of the cross-linked polymer, specifically, of the three-dimensional network structure of the cross-linked polymer.

In addition, when the chelating agent is added during the chopping process, the hydrogel polymer has a three-dimensional network structure in which the main chains formed by polymerizing the water-soluble ethylene-based unsaturated monomer having at least partially neutralized acidic groups are cross-linked by the internal cross-linking agent, and the chelating agent is uniformly dispersed and distributed on the surface of the base resin powder including the hydrogel polymer.

In addition, when the chelating agent is added in both the cross-linking polymerization process and the chopping process, the chelating agent is uniformly dispersed and distributed not only on the surface of the cross-linked polymer in the hydrogel polymer, but also on the surface of the base resin powder including the hydrogel polymer.

When the hydrogel polymer has a three-dimensional network structure as describe above, water retention capacity and absorbency under pressure, which are physical properties of the super absorbent polymer, can be significantly improved compared to the case where the hydrogel polymer has a two-dimensional linear structure that is not additionally cross-linked by an internal cross-linking agent. In addition, the chelating agent uniformly dispersed on the cross-linked polymer or the hydrogel polymer makes it possible to uniformly exhibit excellent antibacterial and deodorizing properties for a long time.

**Step 2**

In the preparation method according to the present disclosure, step 2 is a step of drying and pulverizing the hydrogel polymer prepared in step 1 to form a base resin powder.

The drying method in the drying step is not particularly limited if it has been generally used in the drying process of the hydrogel polymer. Specifically, the drying step may be performed by the method of hot air provision, infrared radiation,

microwave radiation, UV ray radiation, and the like.

When the drying temperature is too low, the drying time may become excessively long and physical properties of the super absorbent polymer to be finally formed may decrease. When the drying temperature is too high, only the surface of the polymer is excessively dried, fine powder may be generated in the subsequent pulverization process, and physical properties of the final super absorbent polymer may decrease. Therefore, the drying of the hydrogel polymer may preferably be performed at a temperature of about 150 to about 200 °C, more preferably at a temperature of 150 °C or more, or 160 °C or more, and 200 °C or less, or 180 °C or less.

Further, the drying time can be appropriately determined depending on the drying temperature. For example, the drying may be performed for 20 to 90 minutes, in consideration of the moisture content of the prepared hydrogel polymer, the drying temperature, and the resulting process efficiency.

The moisture content of the polymer obtained after the drying process under such conditions may be 0.1 to 10 wt% based on the total weight of the dried polymer. That is, the drying method, drying temperature, and drying time can be appropriately selected within the above-described range so that the moisture content of the dried polymer is 0.1 to 10 wt%.

Meanwhile, the preparation method according to the present disclosure may further include the step of coarsely pulverizing the dried hydrogel polymer after the drying process and before the pulverizing process in order to increase the pulverizing efficiency.

The coarse pulverization of the dried hydrogel polymer can be performed using a conventional pulverizing machine. At this time, the pulverizing machine is not particularly limited, and a vertical pulverizer, a turbo cutter, a turbo grinder, a rotary cutter mill, a cutter mill, a disc mill, a shred crusher, a crusher, a chopper, or a disc cutter may be used.

Next, after the drying process or after the coarse pulverizing process, a pulverizing process is performed.

The pulverization may be performed so that the particle diameter of the polymer powder obtained after the pulverization is 150 to 850 $\mu$m. The pulverizing machine used for pulverization to have such a particle diameter is specifically a pin mill, a hammer mill, a screw mill, a roll mill, a disc mill, or a jog mill.

In order to control physical properties of the super absorbent polymer to be prepared as a final product, a step of classifying the polymer particles obtained after the pulverization according to the particle diameter may be performed.

Through this classification process, fines with a particle diameter of less than 150 $\mu$m and coarse particles with a particle diameter of more than 850 $\mu$m are separated and removed, and base resin particles with a diameter of 150 to 850 $\mu$m can be obtained. Additionally, during the classification process, the polymer powder may be classified to have a predetermined weight ratio depending on the particle diameter range.

The classification process can be performed according to conventional methods, such as using an ASTM standard mesh.

The base resin refers to particles or powders formed by drying, pulverizing, and classifying the cross-linked polymer in which a water-soluble ethylene-based unsaturated monomer is cross-linking polymerized using an internal cross-linking agent through the process described above. Specifically, the base resin powder may be in the form of a fine powder having a particle diameter of 150 to 850 $\mu$m.

The base resin manufactured by the preparation method according to the present disclosure has a water retention capacity (CRC) measured according to the EDANA WSP 241.3 of 50 g/g or more, or 55 g/g or more, and 60 g/g or less, or 58 g/ g or less.

In general, the water retention capacity (CRC) of the base resin inevitably decreases in the surface cross-linking reaction to be described later. However, in the present disclosure, as the base resin has a high water retention capacity, the water retention capacity of the final super absorbent polymer can still be maintained high.

In addition, the RM content in the base resin is 500 ppm or less, or 480 ppm, based on the total weight of the base resin, which is lower than that of the base resin manufactured by the conventional preparation method of a super absorbent polymer. Accordingly, the RM content in the final super absorbent polymer can also be maintained low.

In addition, the preparation method according to the present disclosure may further include the step of mixing the base resin powder with a fine reassembly before performing the surface cross-linking process after manufacturing the base resin powder.

In general, when preparing a super absorbent polymer, fine powder is inevitably generated during the pulverization and classification process after drying. Accordingly, in all commercial processes, the generated fine powder is recycled in the form of a fine reassembly with an increased particle size by assembling it with water. Accordingly, in the present disclosure, a fine reassembly reassembled by adding water to the fine powder generated during the preparation of a super absorbent polymer, especially generated during the pulverization and classification process after drying the hydrogel polymer, can be added to the base resin powder, or can be obtained commercially.

The fine reassembly may be mixed in an amount of 20 to 40 parts by weight based on 100 parts by weight of the base resin powder. When the mixing amount of the fine reassembly is excessively high out of the above content range, physical properties of the final super absorbent polymer may be deteriorated.

**Step 3**

In the preparation method according to the present disclosure, step 3 is a step of heat-treating the base resin powder prepared in step 2 in the presence of a surface cross-linking agent to cross-link the surface of the base resin powder to form a surface cross-linked layer.

The surface cross-linking is intended to increase the cross-linking density near the surface of the base resin powder in relation to the cross-linking density inside the particles. Generally, a surface cross-linking agent is applied on the surface of the base resin powder. Accordingly, this reaction occurs on the surface of the base resin powder, which improves crosslinkability on the surface of the particles without substantially affecting the inside of the particles. Therefore, the surface cross-linked base resin powder has a higher degree of cross-linking near the surface than inside, and as a result, it can exhibit more improved absorbency under pressure compared to the case where no surface cross-linked layer is formed. On the other hand, if a surface cross-linked layer is not formed, the absorbency under pressure can be improved by increasing the gel strength. However, when a super absorbent polymer with increased gel strength is applied to an absorbent article without forming a surface cross-linked layer, and pressure is applied to the gel, the gel breaks and microorganisms absorbed in the absorbent article are exposed to the outside, causing secondary contamination. However, when a surface cross-linked layer is formed as described above, the gel shape is well maintained even when pressure is applied to the gel, thereby preventing microorganisms absorbed during use from being exposed to the outside.

Specifically, the surface cross-linking may be performed using a surface cross-linking solution including a surface cross-linking agent and a water solvent.

The surface cross-linking agent is not particularly limited as long as it is a compound capable of reacting with the functional group of the polymer. Preferably, in order to improve properties of the resulting super absorbent polymer, the surface cross-linking agent may be at least one selected from the group consisting of a polyalcohol-based compound; an epoxy compound; a polyamine compound; a haloepoxy compound; a condensation product of a haloepoxy compound; an oxazoline-based compound; a mono-, di- or polyoxazolidinone compound; a cyclic urea compound; a polyvalent metal salt; and an alkylene carbonate-based compound.

Specifically, as the polyalcohol-based compound, at least one selected from the group consisting of ethylene glycol, propylene glycol, 1,3-propanediol, 1,4-butanediol, 1,6-hexanediol, 1,2-hexanediol, 1,3-hexanediol, 2-methyl-1,3-propanediol, 2,5-hexanediol, 2-methyl-1,3-pentanediol, 2-methyl-2,4-pentanediol, tripropylene glycol, and glycerol may be used.

Further, as the epoxy compound, at least one selected from the group consisting of ethylene glycol diglycidyl epoxide, ethylene glycol diglycidyl ether, glycidol and the like may be used. As the polyamine compound, at least one selected from the group consisting of ethylenediamine, diethylenetriamine, triethylenetetraamine, tetraethylenepentamine, pentaethylenehexamine, polyethyleneimine and polyamide polyamine may be used.

Further, as the haloepoxy compound, epichlorohydrin, epibromohydrin, and $\alpha$-methylepichlorohydrin may be used. Meanwhile, as the mono-, di-, or polyoxazolidinone compound, 2-oxazolidinone may be used.

In addition, as the alkylene carbonate-based compound, an alkylene carbonate having 2 to 6 carbon atoms such as ethylene carbonate or propylene carbonate may be used. These may be used alone, or two or more types of alkylene carbonates having different carbon numbers may be mixed and then used. For example, a mixture of ethylene carbonate and propylene carbonate may be used. Additionally, ethylene carbonate and propylene carbonate may be mixed and used at a weight ratio of 2:8 to 8:2, 4:6 to 6:4, or 4.5:5.5 to 5.5:4.5.

In addition, the polyvalent metal salt may specifically be a sulfate or carboxylate including metal such as aluminum, and aluminum sulfate may be more preferably used.

The surface cross-linking agent may be may be added in an amount of 0.001 to 5 parts by weight based on 100 parts by weight of the base resin powder. For example, the surface cross-linking agent may be added in an amount of 0.001 parts by weight or more, 0.005 parts by weight or more, 0.01 parts by weight or more, or 0.1 parts by weight or more and 5 parts by weight or less, 3 parts by weight or less, 2 parts by weight or less, or 1 part by weight or less based on 100 parts by weight of the base resin powder. When adjusting the content of the surface cross-linking agent to the above-mentioned range, a super absorbent polymer exhibiting various physical properties such as excellent absorption performance and permeability can be manufactured. When the content of the surface cross-linking agent is too small, the surface cross-linking reaction hardly occurs. When it exceeds 5 parts by weight based on 100 parts by weight of the base resin powder, absorbency and physical properties may be deteriorated due to excessive surface cross-linking reaction.

The method of mixing the surface cross-linking agent with the base resin powder is not particularly limited. For example, a method of adding the surface cross-linking agent and the base resin powder in a reactor for mixing, a method of spraying the surface cross-linking agent onto the base resin powder, or a method of mixing the base resin powder and the surface cross-linking agent while continuously providing them to a continuously operating mixer may be used.

Water and alcohol may be mixed together in addition to the surface cross-linking agent to be added in the form of a surface cross-linking solution. When adding water and alcohol, there is an advantage in that the surface cross-linking agent can be evenly dispersed in the base resin polymer. At this time, the content of water and alcohol added is about 5 to

about 12 parts by weight based on 100 parts by weight of the polymer for the purpose of inducing even dispersion of the surface cross-linking agent, preventing agglomeration of the base resin powder, and optimizing a surface penetration depth of the surface cross-linking agent.

In addition, the surface cross-linking solution may further include one or more of a glidant, an anti-discoloration agent, a reducing agent, an inorganic filler, aluminum sulfate, and a thickener in addition to the above components.

The glidant acts as a lubricant to improve mixing stability and uniformity when preparing a surface cross-linking solution. In general, permeability among the physical properties of super absorbent polymers has a trade-off relationship with water retention capacity and absorbency under pressure. When the polycarboxylic acid-based copolymer is added, it is possible to provide a super absorbent polymer having excellent permeability while having excellent absorption characteristics such as water retention capacity and absorbency under pressure.

The glidant may specifically include a polycarboxylic acid-based copolymer or a polycarboxylic acid salt, and any one or a mixture of two or more thereof may be used.

Specifically, the polycarboxylic acid-based copolymer may include at least one of a repeating unit represented by the following Chemical Formula 3-a and a repeating unit represented by the following Chemical Formula 3-b:

[Chemical Formula 3-a]

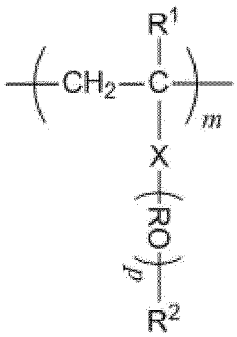

[Chemical Formula 3-b]

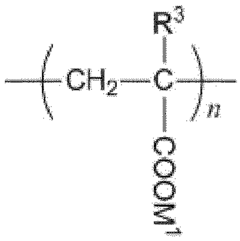

in the Chemical Formulae 3-a and 3-b,

- $R^1$, $R^2$ and $R^3$ are each independently hydrogen or a C1 to C6 alkyl group,
- RO is a C2 to C4 oxyalkylene group,
- $M^1$ is hydrogen, or a monovalent metal or non-metal ion,
- X is -COO-, a C1 to C5 alkyloxy group, or a C1 to C5 alkyldioxy group,
- m is an integer of 1 to 100,
- n is an integer of 1 to 1000, and
- p is an integer of 1 to 150, and when p is 2 or more, two or more repeating -RO-are the same as or different from each other.

Herein, the polycarboxylic acid-based copolymer may include at least one repeating unit having a different structure among the repeating units represented by the Chemical Formula 3-a; at least one repeating unit having a different structure among the repeating units represented by the Chemical Formula 3-b; or the repeating unit represented by the Chemical Formula 3-a and the repeating unit represented by the Chemical Formula 3-b.

More specifically, it may be more advantageous to use a copolymer including a repeating unit derived from an alkoxy polyalkylene glycol mono(meth)acrylic acid ester-based monomer (e.g., methoxy polyethylene glycol monomethacrylate (MPEGMAA), etc.); and a repeating unit derived from (meth)acrylic acid or an ester-based monomer thereof (e.g., (meth)acrylic acid, etc.) as the polycarboxylic acid-based copolymer to achieve the above-described effect. More specifically, it may be more advantageous to use a copolymer including a repeating unit derived from methoxy polyethylene glycol monomethacrylate and a repeating unit derived from (meth)acrylic acid, or a random copolymer including a repeating unit derived from methoxy polyethylene glycol monomethacrylate and a repeating unit derived from (meth)acrylic acid.

In addition, the polycarboxylic acid-based copolymer preferably has a weight average molecular weight of 500 to 1,000,000 g/mol so that the effect of the addition of the polycarboxylic acid-based copolymer is well exhibited. More specifically, the weight average molecular weight may be 500 g/mol or more, 5,000 g/mol or more, 10,000 g/mol or more, 35,000 g/mol or more, or 40,000 g/mol or more, and 1,000,000 g/mol or less, 800,000 g /mol or less, 500,000 g/mol or less, 100,000 g/mol or less, or 60,000 g/mol or less. When the molecular weight of the polycarboxylic acid-based copolymer is less than 500 g/mol, the lubricating action may be reduced, and when it exceeds 1,000,000 g/mol, solubility in water may be reduced.

Meanwhile, in the present disclosure, the weight average molecular weight (Mw) of the polycarboxylic acid-based copolymer can be measured using gel permeation chromatography. Specifically, PL-GPC220 manufactured by Waters was used as the gel permeation chromatography (GPC) instrument, and a Polymer Laboratories PLgel MIX-B column (300 mm in length) was used. An evaluation temperature was 160 °C, and 1,2,4-trichlorobenzene was used for a solvent at a flow rate of 1 mL/min. 10 mg of the polycarboxylic acid-based copolymer sample was pretreated by dissolving in 1,2,4-trichlorobenzene including 0.0125% of BHT at 160 °C for 10 hours using PL-SP260 (manufactured by Agilent Technology) which is a sample pretreatment system, and the sample with a concentration of 10 mg/10 mL was supplied in an amount of 200 μL. Mw was obtained using a calibration curve formed using a polystyrene standard. 9 kinds of the polystyrene standard were used with the molecular weight of 2,000g/mol, 10,000g/mol, 30,000g/mol, 70,000g/mol, 200,000g/mol, 700,000 g/mol, 2,000,000 g/mol, 4,000,000 g/mol, and 10,000,000 g/mol.

Further, specific examples of the polycarboxylic acid salt may include 2-Propenoic acid, 2-methyl, polymer with alpha-(2-methyl-1-oxo-2-propenyl-omega-methoxypoly(oxy-1,2-ethanediyl and 22-Propenoic acid (cas no. 381686-36-8), etc., and a corresponding commercial product such as ACYMA-GK (manufactured by SAN NOPCO KOREA LTD) can also be used.

The glidant may be used in an amount of 1 part by weight or less, more specifically, 0.001 parts by weight or more, 0.03 parts by weight or more, or 0.05 parts by weight or more and 1 part by weight or less, 0.5 parts by weight or less, or 0.1 parts by weight or less based on 100 parts by weight of the base resin powder.

The anti-discoloration agent may include sulfonic acid derivatives such as disodium alpha-sulfoglycolate; and phosphonic acid derivatives such as laurylphosphonic acid or stearylphosphonic acid. Additionally, products such as Blancolen™ HP (manufactured by L. Brugemann KG) may be commercially obtained and included. The anti-discoloration agent may be included in an amount of 0.01 to 0.2 parts by weight, more specifically 0.01 parts by weight or more, 0.03 parts by weight or more, or 0.05 parts by weight or more, and 0.2 parts by weight or less, 0.1 parts by weight or less, or 0.08 parts by weight or less based on 100 parts by weight of the base resin powder.

Further, the reducing agent may include sodium bisulfite, sodium metabisulfite ($Na_2S_2O_5$), sodium hydrosulfite, sodium thiosulfate, or sodium formaldehyde sulfoxylate. The reducing agent may be included in an amount of 0.01 to 0.2 parts by weight based on 100 parts by weight of the base resin powder. More specifically, the reducing agent may be included in an amount of 0.01 parts by weight or more, 0.03 parts by weight or more, or 0.05 parts by weight or more, and 0.2 parts by weight or less, 0.1 parts by weight or less, or 0.08 parts by weight or less based on 100 parts by weight of the base resin powder.

The inorganic filler may include silica, clay, alumina, silica-alumina composite, titania, zinc oxide, or silicate. The inorganic filler may be included in an amount of 0.01 to 0.5 parts by weight based on 100 parts by weight of the base resin powder. More specifically, the inorganic filler may be included in an amount of 0.01 parts by weight or more, and 0.5 parts by weight or less, 0.3 parts by weight or less, or 0.1 parts by weight or less, based on 100 parts by weight of the base resin powder.

Additionally, the surface cross-linking solution may include aluminum sulfate. The aluminum sulfate may be included in an amount of 0.01 to 2 parts by weight based on 100 parts by weight of the base resin powder. More specifically, the aluminum sulfate may be included in an amount of 0.01 parts by weight or more, 0.1 parts by weight or more, or 0.15 parts by weight or more, and 2.0 parts by weight or less, 1 part by weight or less, or 0.5 parts by weight or less, based on 100 parts by weight of the base resin powder.

In addition, the surface cross-linking solution may further include a thickener. When the surface of the base resin powder is further cross-linked in the presence of a thickener, deterioration in physical properties may be minimized even after the pulverization. Specifically, the thickener may be one or more selected from polysaccharides and polymers including hydroxyl groups. More specifically, examples of the polysaccharides include gum-based thickeners and cellulose-based thickeners. Examples of the gum-based thickener include xanthan gum, arabic gum, karaya gum, tragacanth gum, ghatti gum, guar gum, locust bean gum and psyllium seed gum, and examples of the cellulose-based

thickener include hydroxypropylmethylcellulose, carboxymethylcellulose, methylcellulose, hydroxymethylcellulose, hydroxyethylcellulose, hydroxypropylcellulose, hydroxyethylmethylcellulose, hydroxymethylpropylcellulose, hydroxyethylhydroxypropylcellulose, ethylhydroxyethylcellulose, and methylhydroxypropylcellulose. Examples of the polymers including hydroxyl groups include polyethylene glycol and polyvinyl alcohol.

In addition, a surface cross-linking reaction can be performed by heating the base resin powder to which the surface cross-linking agent has been added at a temperature of 120 to 220 °C for 60 to 120 minutes. When the cross-linking temperature is less than 120 °C, the surface cross-linking reaction may not sufficiently occur, and when it exceeds 220 °C, the surface cross-linking reaction may proceed excessively. In addition, when the cross-linking reaction time is too short (less than 60 minutes), the surface cross-linking reaction may not sufficiently occur, and when the cross-linking reaction time exceeds 120 minutes, the cross-linking density on the particle surface becomes too high due to excessive surface cross-linking reaction, which may lead to a decrease in physical properties. More specifically, it can be performed by heating at a temperature of 120 °C or higher, or 180°C or higher, and 220 °C or lower, or 190 °C or lower for 60 minutes or longer, or 80 minutes or longer, and 120 minutes or lower, or 90 minutes or lower. More specifically, the surface cross-linking reaction may be performed at 180 to 190 °C for 80 to 90 minutes.

The heating means for the surface cross-linking reaction is not particularly limited. It is possible to provide a thermal media thereto or provide a heat source directly thereto. At this time, usable thermal media may be a heated fluid such as steam, hot air, hot oil, and the like, but the present invention is not limited thereto. Furthermore, the temperature of the thermal media provided thereto may be properly selected in consideration of the means of the thermal media, heating speed, and target temperature of heating. Meanwhile, an electric heater or a gas heater may be used as the heat source provided directly, but the present disclosure is not limited thereto.

Through the above manufacturing process, a surface cross-linked layer is formed on the surface of the base resin powder.

The preparation method according to the present disclosure may further include a classification process after the surface cross-linking.

The classification process may be performed using an ASTM standard mesh, and as a result, a super absorbent polymer having a particle diameter of 150 to 850 $\mu$m may be obtained.

Specifically, 95 wt% or more, 97 wt% or more, or 99 wt% or more of the super absorbent polymer has a particle diameter of 150$\mu$m to 850$\mu$m. In addition, the super absorbent polymer may include 50 wt% or more of particles having a particle diameter of 300 $\mu$m to 600 $\mu$m. In addition, the super absorbent polymer may include 3 wt% or less, or less than 3 wt% of a fine powder having a particle diameter of less than 150 $\mu$m.

In addition, the super absorbent polymer prepared by the preparation method according to the present disclosure includes a base resin powder including a first cross-linked polymer of a water-soluble ethylene-based unsaturated monomer having at least partially neutralized acidic groups, a persulfate, and a chelating agent; and a surface cross-linked layer formed on the base resin powder and including a second cross-linked polymer in which the cross-linked polymer is further cross-linked using a surface cross-linking agent. The chelating agent may be dispersed in the cross-linked polymer or may be dispersed on the surface of the first cross-linked polymer or base resin powder. As the chelating agent is uniformly dispersed and included in the super absorbent polymer, improved bacterial growth inhibition properties and deodorizing properties can be consistently and safely exhibited without deteriorating physical properties of the super absorbent polymer, such as water retention capacity and absorbency under pressure.

Therefore, according to the present disclosure, there is provided a product including the above-described super absorbent polymer, specifically a sanitary product such as a diaper and a sanitary pad.

The sanitary product may follow the constitution of conventional sanitary products, except that the absorbent material includes the super absorbent polymer of one embodiment.

Hereinafter, the function and effect of the present invention will be described in more detail through specific examples. However, these examples are for illustrative purposes only, and the invention is not intended to be limited by these examples.

**<Preparation of super absorbent polymer>**

**Example 1-1**

In a 3L glass vessel equipped with a stirrer, nitrogen inlet, and thermometer, 438.5 g of acrylic acid was added, and then 1440 ppm of polyethylene glycol diacrylate (PEGDA, Mn=400 g/mol) as an internal cross-linking agent, 80 ppm of bis(2,4,6-trimethylbenzoyl)phenylphosphine oxide as a photo polymerization initiator, 2000 ppm of sodium persulfate (SPS), 8000 ppm of EDTA, 900 ppm of an aqueous solution (concentration of 50%) of ACYMA-GK (manufactured by SAN NOPCO KOREA LTD) as a polycarboxylic acid-based stabilizer, and 98% sodium hydroxide solution were mixed thereto to prepare a monomer composition (solid content (TSC) = 43.3 wt% based on total weight of monomer composition, degree of neutralization = 72%).

The monomer composition was added to a stainless steel container having a width of 250 mm, a length of 250 mm, and a height of 30 mm, and subjected to cross-linking polymerization by irradiating ultraviolet rays for 60 seconds in a UV chamber at 80 °C (irradiation amount: 10 mV/$cm^2$). Then, aging was performed for 2 minutes to obtain a cross-linked polymer. The obtained cross-linked polymer was cut into approximately 5 cm X 5 cm in size, placed in a chopper, and chopped to obtain a hydrogel polymer.

The obtained hydrogel polymer was dried in a hot air oven at 185 °C for 35 minutes, pulverized, and classified using an ASTM standard mesh to obtain base resin (BR) powder with a particle diameter of 150 to 850 $\mu$m (particle diameter of base resin powder: #20/#30/#50/#100/Pan=0/19/64/16/1 (wt%)).

A fine reassembly (CRC 42, particle diameter: #20/#30/#50/#100/Pan=0/19/64/16/1 (wt%)) manufactured by reassembling a fine powder with a particle diameter of less than 150 $\mu$m generated during the drying, pulverization and classification process of the hydrogel polymer with water was added to 100 parts by weight of the base resin powder obtained above, and mixed.

After adding the mixed powder of the base resin powder and fine reassembly prepared above into a surface cross-linking mixer, a surface cross-linking agent in the form of a solution prepared by mixing 4.96 parts by weight of water, 0.5 parts by weight of a mixture of ethylene carbonate (EC) and propylene carbonate (PC) (weight ratio of EC:PC = 1:1), 0.05 parts by weight of ACYMA-GK (manufactured by SAN NOPCO KOREA LTD, aqueous solution with a concentration of 50%), and 0.405 parts by weight of aqueous solution of aluminum sulfate (concentration of 35%) based on 100 parts by weight of the base resin powder was added thereto, and mixed for 30 seconds at 500 rpm.

The resulting mixture was transferred to a jacket-type cylindrical surface reactor equipped with a stirrer, and then a surface cross-linking reaction was performed. The surface cross-linking reaction was performed until the final super absorbent polymer reached the target CRC value of 40 g/g, and the surface cross-linking reaction time was confirmed after completion of the surface cross-linking reaction. In addition, during the surface cross-linking reaction, the temperature in the reactor was raised to a profile of 184 °C after 40 minutes, 181 °C after 60 minutes, and 181 °C after 80 minutes from the start of the surface cross-linking reaction. Afterwards, a further surface cross-linking reaction was performed at 181 °C for 10 minutes.

Thereafter, the surface-treated powder was classified using an ASTM standard mesh to obtain a super absorbent polymer powder with a particle diameter of 150 to 850 $\mu$m.

**Comparative Examples 1-1 to 1-3**

A super absorbent polymer powder was prepared in the same manner as in Example 1-1, except that the conditions described in Table 1 below were followed.

**Example 2-1**

In a 3L glass vessel equipped with a stirrer, nitrogen inlet, and thermometer, 438.5 g of acrylic acid was added, and then 1600 ppm of polyethylene glycol diacrylate (PEGDA, Mn=400 g/mol) as an internal cross-linking agent, 80 ppm of bis(2,4,6-trimethylbenzoyl)phenylphosphine oxide as a photo polymerization initiator, 2000 ppm of sodium persulfate (SPS), and 98% sodium hydroxide solution were mixed thereto to prepare a monomer composition (solid content (TSC) = 41 wt% based on total weight of monomer composition, degree of neutralization = 72%).

The monomer composition was added to a stainless steel container having a width of 250 mm, a length of 250 mm, and a height of 30 mm, and subjected to cross-linking polymerization by irradiating ultraviolet rays for 60 seconds in a UV chamber at 80 °C (irradiation amount: 10 mV/$cm^2$). Then, aging was performed for 2 minutes to obtain a cross-linked polymer. The obtained cross-linked polymer was cut into approximately 5 cm $\times$ 5 cm in size, placed in a chopper, and chopped to obtain a hydrogel polymer. During the chopping, an aqueous solution of EDTA-4Na (concentration: 40%) was added such that EDTA-4Na was added in an amount of 8000 ppm based on the total weight of the acrylic acid monomer.

The obtained hydrogel polymer was dried in a hot air oven at 185 °C for 35 minutes, pulverized, and classified using an ASTM standard mesh to obtain base resin (BR) powder with a particle diameter of 150 to 850 $\mu$m (particle diameter of base resin powder: #20/#30/#50/#100/Pan=0/19/64/16/1 (wt%)).

A fine reassembly (CRC 42, particle diameter: #20/#30/#50/#100/Pan=0/19/64/16/1 (wt%)) manufactured by reassembling a fine powder with a particle diameter of less than 150 $\mu$m generated during the drying and pulverization process with water was added to 100 parts by weight of the base resin powder obtained above, and mixed.

A surface cross-linking solution prepared by mixing 4.7 parts by weight of water, 0.2 parts by weight of ethylene carbonate, 0.05 parts by weight of ACYMA-GK (manufactured by SAN NOPCO KOREA LTD, aqueous solution with a concentration of 50%), and 0.35 parts by weight of aqueous solution of aluminum sulfate (concentration of 35%) based on 100 parts by weight of the base resin powder was added to the mixed powder of the base resin powder and fine reassembly prepared above, and mixed for 30 seconds at 500 rpm.

The resulting mixture was transferred to a jacket-type cylindrical surface reactor equipped with a stirrer, and then

a surface cross-linking reaction was performed. The surface cross-linking reaction was performed until the final super absorbent polymer reached the target CRC value of 40 g/g, and the surface cross-linking reaction time was confirmed after completion of the surface cross-linking reaction. In addition, during the surface cross-linking reaction, the temperature in the reactor was raised to a profile of 184 °C after 40 minutes, 181 °C after 60 minutes, and 181 °C after 80 minutes from the start of the surface cross-linking reaction. Afterwards, a further surface cross-linking reaction was performed at 181 °C for 10 minutes.

Thereafter, the surface-treated powder was classified using an ASTM standard mesh to obtain a super absorbent polymer powder with a particle diameter of 150 to 850 $\mu$m.

**Comparative Examples 2-1 to 2-3**

A super absorbent polymer was prepared in the same manner as in Example 2-1, except that the cross-linking polymerization and surface cross-linking reaction were performed under the conditions shown in Table 2 below.

**Example 2-2, Example 2-3 and Comparative Example 2-4**

A super absorbent polymer was prepared in the same manner as in Example 2-1, except that a surface cross-linking solution prepared by mixing 4.96 parts by weight of water, 0.5 parts by weight of a mixture of ethylene carbonate and propylene carbonate (weight ratio of EC:PC = 1:1), 0.05 parts by weight of aqueous solution (concentration of 50%) of ACYMA-GK (manufactured by SAN NOPCO KOREA LTD), and 0.405 parts by weight of aqueous solution of aluminum sulfate (concentration of 35%) based on 100 parts by weight of the base resin powder was used; and the polymerization and surface cross-linking reaction were performed under the conditions shown in Table 2 below.

**Example 3**

In a 3L glass vessel equipped with a stirrer, nitrogen inlet, and thermometer, 438.5 g of acrylic acid was added, and then 1600 ppm of polyethylene glycol diacrylate (PEGDA, Mn=400 g/mol) as an internal cross-linking agent, 80 ppm of bis(2,4,6-trimethylbenzoyl)phenylphosphine oxide as a photo polymerization initiator, 2000 ppm of sodium persulfate (SPS), 4000 ppm of EDTA, 900 ppm of an aqueous solution (concentration of 50%) of ACYMA-GK (manufactured by SAN NOPCO KOREA LTD) as a polycarboxylic acid-based stabilizer, and 98% sodium hydroxide solution were mixed thereto to prepare a monomer composition (solid content (TSC) = 41 wt% based on total weight of monomer composition, degree of neutralization = 72%).

The monomer composition was added to a stainless steel container having a width of 250 mm, a length of 250 mm, and a height of 30 mm, and subjected to cross-linking polymerization by irradiating ultraviolet rays for 60 seconds in a UV chamber at 80 °C (irradiation amount: 10 $mV/cm^2$). Then, aging was performed for 2 minutes to obtain a cross-linked polymer. The obtained cross-linked polymer was cut into approximately 5 cm $\times$ 5 cm in size, placed in a chopper, and chopped to obtain a hydrogel polymer. During the chopping, an aqueous solution of EDTA-4Na (concentration: 40%) was added such that EDTA-4Na was added in an amount of 4000 ppm based on the total weight of the acrylic acid monomer.

The obtained hydrogel polymer was dried in a hot air oven at 185 °C for 35 minutes, pulverized, and classified using an ASTM standard mesh to obtain base resin (BR) powder with a particle diameter of 150 to 850 $\mu$m (particle diameter of base resin powder: #20/#30/#50/#100/Pan=0/19/64/16/1 (wt%)).

A fine reassembly (CRC 42, particle diameter: #20/#30/#50/#100/Pan=0/19/64/16/1 (wt%)) manufactured by reassembling a fine powder with a particle diameter of less than 150 $\mu$m generated during the drying, and pulverization process with water was added to 100 parts by weight of the base resin powder obtained above, and mixed.

After adding the mixed powder of the base resin powder and fine reassembly prepared above into a surface cross-linking mixer, a surface cross-linking solution prepared by mixing 4.7 parts by weight of water, 0.2 parts by weight of ethylene carbonate 0.05 parts by weight of ACYMA-GK (manufactured by SAN NOPCO KOREA LTD, aqueous solution with a concentration of 50%), and 0.35 parts by weight of aqueous solution of aluminum sulfate (concentration of 35%) based on 100 parts by weight of the base resin powder was added thereto, and mixed for 30 seconds at 500 rpm.

The resulting mixture was transferred to a jacket-type cylindrical surface reactor equipped with a stirrer, and then a surface cross-linking reaction was performed. The surface cross-linking reaction was performed until the final super absorbent polymer reached the target CRC value of 40 g/g, and the surface cross-linking reaction time was confirmed after completion of the surface cross-linking reaction. In addition, during the surface cross-linking reaction, the temperature in the reactor was raised to a profile of 184 °C after 40 minutes, 181 °C after 60 minutes, and 181 °C after 80 minutes from the start of the surface cross-linking reaction. Afterwards, a further surface cross-linking reaction was performed at 181 °C for 10 minutes.

Thereafter, the surface-treated powder was classified using an ASTM standard mesh to obtain a super absorbent polymer powder with a particle diameter of 150 to 850 $\mu$m.

**Experimental Example 1**

CRC was measured for the base resin powder prepared during the manufacturing process of the super absorbent polymer of Examples and Comparative Examples by the following method, and the results are shown in the table below.

Unless otherwise indicated, all evaluations of the following physical properties were conducted at room temperature (25 °C), and physiological saline or saline refers to 0.9 wt% aqueous solution of sodium chloride (NaCl).

(1) Centrifuge retention capacity (CRC)

Those having a particle diameter of 150 $\mu$m to 850 $\mu$m were taken from the base resin powder, and centrifuge retention capacity (CRC) by water absorption capacity under a non-loading condition was measured according to the EDANA WSP 241.3.

Specifically, polymers classified through a sieve of 150 $\mu$m to 850 $\mu$m from the base resin powder obtained in one of Examples and Comparative Examples were prepared. After inserting $W_0$ (g, about 0.2 g) of the polymer uniformly in a nonwoven fabric envelope and sealing the same, it was soaked in saline (0.9 wt%) at room temperature. After 30 minutes, the envelope was centrifuged at 250G for 3 minutes to drain, and the weight $W_2$ (g) of the envelope was measured. Further, after carrying out the same operation without using the polymer, the weight $W_1$ (g) of the envelope was measured. Then, CRC (g/g) was calculated by using the obtained weight values according to the following Equation 3.

[Equation 3]

$$CRC\ (g/g) = \{[W_2(g) - W_1(g)]/W_0(g)\} - 1$$

in Equation 3,

$W_0$ (g) is an initial weight (g) of the base resin,
$W_1$ (g) is a weight of an envelope measured after immersing the envelope in physiological saline for 30 minutes for absorption, followed by dehydration with 250 G for 3 minutes using a centrifuge without using the base resin, and
$W_2$ (g) is a weight of an envelope including a super absorbent polymer measured after immersing the base resin in physiological saline at room temperature for 30 minutes for absorption, followed by dehydration with 250 G for 3 minutes using a centrifuge.

**Experimental Example 2**

The effect of the amount of the chelating agent added during the preparation of a super absorbent polymer on antibacterial activity of the super absorbent polymer was evaluated.

Specifically, a super absorbent polymer was prepared in the same manner as in Example 1-1, except that EDTA-4Na was added at 0, 3000, 4000, 5000, 5300, 8000, and 13000 ppm based on the total weight of acrylic acid instead of EDTA, respectively.

The antibacterial properties of the prepared super absorbent polymer were evaluated by the following method.

50 ml of synthetic urine inoculated with 3000 CFU/ml of Proteus Mirabilis (ATCC 29906) was added to 2 g of the super absorbent polymer with no EDTA, and then incubated in an incubator at 35 °C for 12 hours. The incubated sample was washed with 150 mL of saline (0.9 wt% sodium chloride aqueous solution), and incubated on a Nutrient broth agar (BD DIFCO.) to measure CFU (Colony Forming Unit; CFU/ml) as the physical properties of the control group.

In addition, 50 ml of synthetic urine inoculated with 3000 CFU/ml of Proteus Mirabilis (ATCC 29906) was added to 2 g of each super absorbent polymer prepared by varying the amount of EDTA-4Na added above, and then incubated in an incubator at 35 °C for 12 hours. 150 mL of saline (0.9 wt% sodium chloride aqueous solution) was added to the incubated sample, and shaken for 1 minute to mix evenly. The resulting diluted solution was spread on a Nutrient broth agar (BD DIFCO.) plate, and incubated in an incubator at 30 °C for 24 hours to measure CFU (Colony Forming Unit; CFU/ml) as the physical properties of the experimental group.

The bacterial growth inhibition rate defined by Equation 1 below was calculated using these measurement results, and antibacterial activity of super absorbent polymer was evaluated according to the amount of EDTA-4Na added.

Proteus Mirabilis, the strain used to measure the bacterial growth inhibition rate, secretes urease, which decomposes urea in urine and generates ammonia, which causes bad odor. Therefore, the antibacterial and deodorizing effects of super absorbent polymer can be properly evaluated with the Proteus Mirabilis bacterial growth inhibition rate. However, if the proliferation of Proteus Mirabilis is excessively suppressed for the deodorizing effect, it may kill other

beneficial bacteria and cause problems such as skin rashes, so appropriate suppression of proliferation is necessary.

[Equation 1]

$$\text{Bacterial growth inhibition rate} = [1- \{CFU(12h)/CFU\ control(12h)\}] \times 100\ (\%)$$

in Equation 1,

CFU(12 h) represents the number of individuals of proliferated bacteria per unit volume of synthetic urine (CFU/ml), which was obtained by adding 2 g of the super absorbent polymer of an experimental group prepared by adding the chelating agent (EDTA-Na) to 50 ml of synthetic urine inoculated with 3000 CFU/ml of bacteria of Proteus mirabilis (ATCC 29906), and then incubating at 35 °C for 12 hours, and
CFU control(12 h) represents the number of individuals of proliferated bacteria per unit volume of synthetic urine (CFU/ml), which was obtained by incubating synthetic urine inoculated with the above bacteria on the super absorbent polymer of a control group prepared without using a chelating agent (EDTA) under the same conditions, representing the number of individuals of bacteria per unit volume of synthetic urine (CFU/ml) measured for the control group.

Meanwhile, the synthetic urine used in Experimental Example was prepared by the following method.

<Manufacture of synthetic urine>

1. Preparation of stock solution

A stock solution was prepared by mixing the following compounds, and sterilized under high pressure at 120 °C for 15 minutes before use.

sodium chloride (NaCl)(Extra pure (99.0 or more), manufactured by Daejung) 8.78g
dipotassium hydrogen phosphate ($K_2HPO_4$) (Extra pure (98.0 or more), manufactured by Duksan) 4.48g
sodium dihydrogen phosphate ($NaH_2PO_4$) (Extra pure (99.0 or more) manufactured by Daejung) 1.2g
ammonium chloride ($NH_4Cl$) Extra pure (99.0 or more), manufactured by Daejung) 6.68g
disodium sulfate decahydrate ($Na_2SO_4$) (Extra pure (98.0 or more) 6.44g
lactic acid ($C_3H_6O_3$) (Extra pure (90.0 or more), manufactured by Daejung) 0.45g
yeast extract (manufactured by BD Bacto) 20g
distilled water (quadruple distilled water) 1000 mL

2. Preparation of urea solution

The following compounds were mixed, and filtered through a 0.22 μm filter to prepare a urea solution.

Urea ($NH_2CONH_2$) (BioReagent (98% or more), manufactured by Sigma-Aldrich) 36g
D-glucose ($C_6H_{12}O_6$) (ACS Reagent, manufactured by Sigma-Aldrich) 0.18 g
distilled water (quadruple distilled water) 100 mL

3. Preparation of cationic solution

A cationic solution was prepared by mixing the following compounds, and sterilized under high pressure at 120 °C for 15 minutes before use.

magnesium chloride hexahydrate ($MgCl_2.6H_2O$)(Extra pure (98.0 or more), manufactured by Duksan) 1.22g
calcium chloride dihydrate ($CaCl_2.2H_20$)(Extra pure (98.0 or more), manufactured by Daejung) 0.67g
distilled water (quadruple distilled water) 20 mL

4. Preparation of synthetic urine

Synthetic urine was prepared by mixing 940 g of the stock solution, 50 ml of the urea solution, and 10 ml of the cation solution prepared in steps 1 to 3 above. When calculated from the amounts of raw materials of NaCl, urea, and polyvalent metal used in the preparation of synthetic urine, the NaCl content was about 0.8 wt%, the urea content was

about 1.8 wt%, and the polyvalent metal content was about 0.095 wt% based on the total weight of the prepared synthetic urine.

The experimental results are shown in FIG. 1.

FIG. 1 is a graph showing the results of measuring the bacterial growth inhibition rate according to the amount of chelating agent (EDTA-Na) added.

**Experimental Example 3**

The physical properties of the super absorbent polymer prepared in one of Examples and Comparative Examples were evaluated in the following manner, and the results are shown in the table below.

Unless otherwise indicated, all evaluations of the following physical properties were conducted at room temperature (25 °C), and physiological saline or saline refers to 0.9 wt% aqueous solution of sodium chloride (NaCl).

(1) Centrifuge retention capacity (CRC)

Centrifuge retention capacity (CRC) by water absorption capacity under a non-loading condition was measured according to the EDANA WSP 241.3 in the same manner as in (1) of Experimental Example 1, except that the super absorbent polymer prepared in one of Examples and Comparative Examples was used instead of the base resin powder.

Specifically, polymers classified through a sieve of #30-50 from the super absorbent polymer obtained in one of Examples and Comparative Examples were prepared. After inserting $W_0$ (g, about 0.2 g) of the polymer uniformly in a nonwoven fabric envelope and sealing the same, it was soaked in saline (0.9 wt%) at room temperature. After 30 minutes, the envelope was centrifuged at 250G for 3 minutes to drain, and the weight $W_2$ (g) of the envelope was measured. Further, after carrying out the same operation without using the polymer, the weight $W_1$ (g) of the envelope was measured. Then, CRC (g/g) was calculated by using the obtained weight values according to the following Equation 3.

[Equation 3]

$$CRC\ (g/g) = \{[W_2(g) - W_1(g)]/W_0(g)\} - 1$$

in Equation 3,

$W_0$ (g) is an initial weight (g) of the super absorbent polymer,
$W_1$ (g) is a weight of an envelope measured after immersing the envelope in physiological saline for 30 minutes for absorption, followed by dehydration with 250 G for 3 minutes using a centrifuge without using the super absorbent polymer, and
$W_2$ (g) is a weight of an envelope including a super absorbent polymer measured after immersing the super absorbent polymer in physiological saline at room temperature for 30 minutes for absorption, followed by dehydration with 250 G for 3 minutes using a centrifuge.

(2) Absorbency under pressure (AUP)

The absorbency under pressure at 4826 Pa (0.7 psi) of each polymer was measured according to the EDANA WSP 242.3-10. When measuring absorbency under pressure, the classified polymer used in the above CRC measurement was used.

A 400 mesh stainless steel screen was installed in a cylindrical bottom of a plastic having an inner diameter of 25 mm. W0 (g, 0.16 g) of the super absorbent polymer was uniformly scattered on the screen at room temperature and a humidity of 50%. Thereafter, a piston which can uniformly provide a load of 4826 Pa (0.7 psi) was placed thereon. Herein, the outer diameter of the piston was slightly smaller than 25 mm, there was no gap with the inner wall of the cylinder, and jig-jog of the cylinder was not interrupted. At this time, the weight W3 (g) of the device was measured.

Subsequently, a glass filter having a diameter of 90 mm and a thickness of 5 mm was placed in a petri dish having a diameter of 150 mm, and saline composed of 0.9 wt% sodium chloride was poured in the dish. At this time, the saline was poured until the surface level of the saline became equal to the upper surface of the glass filter. One sheet of filter paper with a diameter of 90 mm was placed thereon. After the measuring device was placed on the filter paper, the liquid was absorbed for 1 hour under a load. After 1 hour, the measuring device was lifted, and the weight $W_4$ (g) was measured.

Then, absorbency under pressure (g/g) was calculated by using the obtained weight values according to the following Equation.

[Equation 4]

$$AUP(g/g) = [W_4(g) - W_3(g)]/W_0(g)$$

(3) EFFC (Arithmetic average of centrifuge retention capacity and absorbency under pressure)

EFFC of the super absorbent polymer prepared in one of Examples and Comparative Examples was calculated according to Equation 2 below.

[Equation 2]

$$EFFC = (CRC + AUP)/2$$

in Equation 2,

CRC is centrifuge retention capacity of the super absorbent polymer to saline (0.9 wt% aqueous solution of sodium chloride) for 30 minutes measured according to the EDANA WSP 241.3, and
AUP is absorbency under pressure of the super absorbent polymer to saline (0.9 wt% aqueous solution of sodium chloride) at 4826 Pa (0.7 psi) for 1 hour measured according to the EDANA WSP 242.3-10.

(4) Residual monomers (RM, ppm)

The residual monomer content of the super absorbent polymer prepared in one of Examples and Comparative Examples was calculated according to the EDANA WSP 210.2.

Specifically, 1.000 g of a super absorbent polymer powder sample with a particle diameter of 150 to 850 μm and 184.3 g of 0.90 wt% aqueous solution of sodium chloride were placed in a 250 ml plastic container with a lid and stirred for 1 hour to extract unreacted acrylic acid. The extracted solution was analyzed using HPLC/CAD under the following conditions, and the content of unreacted acrylic acid, which is a residual monomer, was measured (ppm, based on total weight of super absorbent polymer).

<Analysis conditions>

* Mobile phase

- Mobile phase A: 1000 mL of acetonitrile was filtered using a solvent clarification system to remove foreign substances.
- Mobile phase B: 1000 mL of ultrapure water was filtered using a solvent clarification system to remove foreign substances.

* HPLC analysis conditions

- Column: Capcellpak C18 (4.6 mm I.D. × 50 mm L., 3 μm, Shiseido)
- Eluent: Mobile phase A/ Mobile phase B= 75/25 (v/v, %)
- Flow rate: 1 mL/min
- Column temp.: 40 °C
- Run time: 15 min
- Injection Volume: 10 μℓ

* CAD analysis conditions

- Gas pressure=232,353 Pa (33.7 psi), Corona=normal
- Total flow=1.14 mL/min, Flow ratio=0.48
- The measurement was performed after measuring at 100 pA, followed by adjusting the range according to the content.

(The size of the peak increases from 100 pA to 50 pA to 20 pA.)

(5) Hunter Color (B)

The b value was measured using a colorimeter (Color Quest II, Hunter Lab Co.).

L, a, and b measured by a colorimeter represent the values on coordinate axes representing unique colors. 'L' represents whiteness or brightness, and can have values from 0 to 100. As the 'L' value approaches 0, it represents black, and as it approaches 100, it represents white. 'a' can have positive and negative values relative to 0, where a higher value above 0 indicates a redder color and a lower value below 0 indicates a greener color. 'b' can also have positive and negative values relative to 0, where a higher value above 0 indicates a yellowish color, and a lower value below 0 indicates a bluish color.

(6) Antibacterial activity

Antibacterial activity against Proteus Mirabilis (ATCC 29906) was evaluated in the same manner as in Experimental Example 2, except that the super absorbent polymer prepared in one of Examples and Comparative Examples was used instead of the super absorbent polymer of the experimental group.

(7) Analysis of chelating agent content

The chelating agent content of the super absorbent polymer prepared in one of Examples and Comparative Examples was measured according to the method described below (ppm, based on total weight of super absorbent polymer).

<Analysis method>

1. Extraction

Method type: Preparation method - solvent extraction

1) Each sample was prepared by adding 100 mL of 0.9% NaCl aqueous solution to 0.5 g of the super absorbent polymer prepared in Example or Comparative Example and extracting EDTA-4Na.

2. HPLC/UV analysis

For HPLC/UV analysis, Waters 1525 Binary HPLC Pump (manufactured by Waters) and Waters 2489 UV/Visible Detector (manufactured by Waters) were used.

2.1 Method type: Measurement method - quantitative analysis

1) A device was stabilized, a standard sample was injected, and a calibration curve was created. At this time, concentration samples prepared by dissolving EDTA in 100 mL of 0.9% NaCl aqueous solution at a concentration of 1000, 3000, 4000, 5000, 6000, 7000, 8000, 10000, and 13000 ppm were used as the standard samples.
2) The sample prepared in step 1) of 1. Extraction above was injected into the device, and the content of EDTA, a chelating agent, was calculated using the calibration curve.
3) HPLC conditions

- Column: PRP-1 (manufactured by Hamilton) (4.6 mm ID × 150 mm L, 5 μm)
- UV Detection wavelength: 210 nm
- Mobile phase: 3.42 g of $H_3PO_4$ + 4L of $H_2O$ (85%), pH =2.5
- Gradient elution: 0.0074 mol of $H_3PO_4$
- Flow rate: 0.8 mL/min
- Column temp.: 40 °C
- Injection volume: 10 μL
- Run time: 20 min.

FIG. 2 is a graph showing the results of observing the change in the amount of chelating agent detected in the super absorbent polymer according to the amount of chelating agent added during the preparation of the super absorbent polymer through HPLC analysis.

[Table 1]

| | | Comparative Example 1-1 | Comparative Example 1-2 | Comparative Example 1-3 | Example 1-1 |
|---|---|---|---|---|---|
| Preparation of hydrogel polymer (adding chelating agent during cross-linking polymerizati on) | SPS (ppm[a]) | 2000 | 2000 | 2000 | 2000 |
| | EDTA (ppm[a]) | 0 | 3000 | 5000 | 8000 |
| Adding chelating agent during chopping | EDTA (ppm[a]) | 0 | 0 | 0 | 0 |
| Weight ratio of SPS:EDTA | | - | 1:1.5 | 1:2.5 | 1:4 |
| Surface cross-linking | Surface cross-linking reaction time (min) | 80 | 90 | 70 | 90 |
| BR physical properties | CRC (g/g) | 56.4 | 55.5 | 54.6 | 54.4 |
| SAP final physical properties | EDTA content (ppm[b]) | 0 | 2300 | 3800 | 6000 |
| | CRC (g/g) | 40.4 | 40.6 | 40.3 | 40.7 |
| | 0.7 AUP (g/g) | 19.5 | 20.4 | 20.6 | 20.9 |
| | EFFC | 30.0 | 30.5 | 30.5 | 30.8 |
| | RM (ppm) | 376 | 391 | 472 | 446 |
| | Hunter Color B | 4.4 | 7.8 | 7.5 | 7.4 |
| | CFU/ml | 14,000,000,00 0 | 7,600,000,00 0 | 1,900,000,00 0 | 81,000,000 |
| | Bacterial growth inhibition rate (%) | 0 | 45.7 | 86.4 | 99.4 |

[Table 2]

| | | Compa rative Examp le 2-1 | Compa rative Examp le 2-2 | Compa rative Examp le 2-3 | Compa rative Examp le 2-4 | Examp le 2-1 | Examp le 2-2 | Examp le 2-3 | Examp le 3 |
|---|---|---|---|---|---|---|---|---|---|
| Preparation of hydrogel polymer (adding chelating agent during cross- linking polymerization ) | SPS (ppm[a]) | 1400 | 1400 | 4200 | 2000 | 2800 | 2000 | 2000 | 2000 |
| | EDTA (ppm[a]) | 0 | 0 | 0 | 0 | 0 | 0 | 0 | 4000 |
| Adding chelating agent during chopping | EDTA (ppm[a]) | 0 | 8000 | 8000 | 5300 | 8000 | 8000 | 13000 | 4000 |
| Weight ratio of SPS:EDTA | | - | 1:5.71 | 1:1.90 | 1:2.65 | 1:2.86 | 1:4 | 1:6.5 | 1:4 |
| Surface cross-link- ing | Surface cross-link- ing reac- tion time (min) | 100 | 90 | 70 | 90 | 90 | 90 | 70 | 90 |
| BR physical properties | CRC (g/g) | 58.8 | 54.9 | 49.5 | 55.0 | 53.4 | 53.1 | 52.1 | 53.2 |
| SAP final physical properties | EDTA content (ppm[b]) | 0 | 6000 | 6000 | 4000 | 6000 | 6000 | 10000 | 6000 |
| | CRC (g/g) | 39.7 | 39.8 | 39.6 | 39.5 | 40.3 | 39.9 | 39.7 | 39.9 |
| | 0.7 AUP (g/g) | 22.6 | 22.5 | 18.5 | 22 | 21.8 | 23 | 21.2 | 23.1 |
| | EFFC | 31.2 | 31.2 | 29.1 | 30.8 | 31.4 | 31.5 | 30.5 | 31.5 |
| | RM (ppm) | 390 | 502 | 275 | 370 | 376 | 347 | 363 | 350 |
| | Hunter Color B | 6.7 | 5.9 | 10.6 | 8.5 | 8.7 | 8.1 | 7.2 | 8.3 |
| | CFU/ml | 20,000 ,000,0 00 | 85,000 ,000 | 83,000 ,000 | 2,000, 000,00 0 | 83,000 ,000 | 85,000 ,000 | 55,000 ,000 | 85,000 ,000 |

(continued)

| | | Compa rative Examp le 2-1 | Compa rative Examp le 2-2 | Compa rative Examp le 2-3 | Compa rative Examp le 2-4 | Examp le 2-1 | Examp le 2-2 | Examp le 2-3 | Examp le 3 |
|---|---|---|---|---|---|---|---|---|---|
| | Bacterial growth in-hibition rate (%) | 0 | 99.6 | 99.6 | 90.0 | 99.6 | 99.6 | 99.7 | 99.6 |

In Tables 1 and 2, 'ppm' of a is based on the total weight of acrylic monomer (AA), and 'ppm' of b is based on the total weight of the finally prepared super absorbent polymer (SAP).

As a result of the experiment, when the chelating agent was not used in the preparation of the super absorbent polymer, or even when the chelating agent was added but the amount added was too small out of the range defined in the present disclosure, the antibacterial effect was greatly reduced (Comparative Examples 1-1, 1-2, 1-3, 2-1, and 2-4). Meanwhile, as the amount of chelating agent added increased, the antibacterial effect increased. However, when the amount added exceeded a certain amount, the increase in antibacterial effect compared to the amount added was not large, and absorption performance of the super absorbent polymer, especially EFFC, decreased due to the excessive chelating agent. Accordingly, it was confirmed that the amount of chelating agent added must be controlled in order to fully realize the effect of improving the antibacterial effect while maintaining excellent absorption performance.

In addition, when a persulfate was added at an excessively high amount out of the range defined in the present disclosure, the Hunter color B value of the super absorbent polymer increased significantly (Comparative Example 2-3). On the other hand, when the persulfate was added at an excessively low amount out of the range defined in the present disclosure, the residual monomer content significantly increased (Comparative Example 2-2).

## Claims

**1.** A super absorbent polymer comprising

a base resin powder comprising a first cross-linked polymer of a water-soluble ethylene-based unsaturated monomer having at least partially neutralized acidic groups, and a chelating agent; and
a surface cross-linked layer formed on the base resin powder and comprising a second cross-linked polymer in which the first cross-linked polymer is further cross-linked using a surface cross-linking agent,
wherein the following conditions (i) to (iii) are satisfied:

(i) CFU(12h), which is the number of individuals of proliferated bacteria per unit volume of synthetic urine (CFU/ml) obtained by adding 2 g of the super absorbent polymer to 50 ml of synthetic urine inoculated with 3000 CFU/ml of bacteria of Proteus mirabilis (ATCC29906), and then incubating at 35 °C for 12 hours: $10^9$ or less
(ii) Residual monomer content measured according to the EDANA WSP 210.3: 450 ppm or less
(iii) Hunter color B: 10 or less.

**2.** The super absorbent polymer of Claim 1,

wherein the super absorbent polymer has a bacterial growth inhibition rate represented by the following Equation 1 of 95% or more:

[Equation 1]

$$\text{Bacterial growth inhibition rate} = [1- \{CFU(12h) / CFU\ control(12h)\}]*100\ (\%)$$

in Equation 1, CFU(12 h) represents the number of individuals of proliferated bacteria per unit volume of synthetic urine (CFU/ml), which was obtained by adding 2 g of the super absorbent polymer to 50 ml of synthetic urine inoculated with 3000 CFU/ml of bacteria of Proteus mirabilis (ATCC29906), and then incubating at 35 °C for 12 hours, and
CFU control (12 h) represents the number of individuals of proliferated bacteria per unit volume of synthetic urine (CFU/ml), which was obtained by incubating synthetic urine inoculated with the above bacteria on the super absorbent polymer of a control group under the same conditions, wherein the super absorbent polymer of the control group refers to the same super absorbent polymer except that the base resin powder does not comprise a chelate.

**3.** The super absorbent polymer of Claim 1,
wherein the super absorbent polymer further satisfies the following conditions (iv) to (vi):

(iv) Centrifuge retention capacity (CRC) measured according to the EDANA WSP 241.2: 39 g/g or more
(v) Absorbency under pressure (AUP) at 4826 Pa (0.7 psi) measured according to the EDANA WSP 242.3-10: 17

g/g or more
(vi) Arithmetic average of centrifuge retention capacity and absorbency under pressure (EFFC) represented by the following Equation 2: 28 or more

$$[Equation\ 2]\ EFFC = (CRC + AUP)/2$$

in Equation 2, CRC is centrifuge retention capacity of the super absorbent polymer to saline (0.9 wt% aqueous solution of sodium chloride) for 30 minutes measured according to the EDANA WSP 241.3, and AUP is absorbency under pressure of the super absorbent polymer to saline (0.9 wt% aqueous solution of sodium chloride) at 4826 Pa (0.7 psi) for 1 hour measured according to the EDANA WSP 242.3-10.

**4.** The super absorbent polymer of Claim 1,
wherein the chelating agent is dispersed inside or on a surface of the base resin powder.

**5.** The super absorbent polymer of Claim 1,
wherein the chelating agent is at least one selected from the group consisting of ethylenediamine tetraacetic acid, diethylenetriamine pentaacetic acid, phenolic acid, citric acid, amino acid, and a salt thereof.

**6.** The super absorbent polymer of Claim 1,
wherein the chelating agent is comprised in an amount of 4,500 to 12,000 ppm based on a total weight of the super absorbent polymer.

**7.** The super absorbent polymer of Claim 1,
wherein the base resin powder further comprises a persulfate.

**8.** The super absorbent polymer of Claim 7,
wherein the persulfate comprises one or more of sodium persulfate and potassium persulfate.

**9.** A preparation method of the super absorbent polymer according to Claim 1, comprising the steps of:

forming a hydrogel polymer by performing cross-linking polymerization of a water-soluble ethylene-based unsaturated monomer having at least partially neutralized acidic groups in the presence of an internal cross-linking agent and a persulfate, followed by chopping;
forming a base resin powder by drying and pulverizing the hydrogel polymer; and
cross-linking a surface of the base resin powder by heat treating the base resin powder in the presence of a surface cross-linking agent,
wherein the persulfate is added in an amount of 1,800 to 3,500 ppm based on a total weight of the water-soluble ethylene-based unsaturated monomer, and
a chelating agent is additionally added in an amount of 6,000 to 15,000 ppm based on a total weight of the water-soluble ethylene-based unsaturated monomer in at least one process of the cross-linking polymerization and chopping.

**10.** The preparation method of the super absorbent polymer according to Claim 9,
wherein the persulfate comprises one or more of sodium persulfate and potassium persulfate.

**11.** The preparation method of the super absorbent polymer according to Claim 9,
wherein the chelating agent is at least one selected from the group consisting of ethylenediamine tetraacetic acid, diethylenetriamine pentaacetic acid, phenolic acid, citric acid, amino acid, and a salt thereof.

**12.** The preparation method of the super absorbent polymer according to Claim 9,
wherein the additional addition of the chelating agent is performed during the cross-linking polymerization, and a weight ratio of the persulfate and the chelating agent is 1:3 to 1:4.

**13.** The preparation method of the super absorbent polymer according to Claim 9,
wherein the additional addition of the chelating agent is performed during chopping, and a weight ratio of the persulfate and the chelating agent is 1:2 to 1:7.

**14.** The preparation method of the super absorbent polymer according to Claim 9,
wherein the chopping is performed using a chopper.

**15.** The preparation method of the super absorbent polymer according to Claim 9,
wherein the surface cross-linking agent is at least one selected from the group consisting of a polyalcohol-based compound; an epoxy compound; a polyamine compound; a haloepoxy compound; a condensation product of a haloepoxy compound; an oxazoline-based compound; a mono-, di- or polyoxazolidinone compound; a cyclic urea compound; a polyvalent metal salt; and an alkylene carbonate-based compound.

**16.** The preparation method of the super absorbent polymer according to Claim 9,
wherein the preparation method further comprises the step of mixing a fine reassembly in an amount of 20 to 40 parts by weight based on 100 parts by weight of the base resin powder, before surface cross-linking.

**17.** A product comprising the super absorbent polymer according to Claim 1.

**18.** The product according to Claim 17,
wherein the product is a sanitary product.

【FIG. 1】

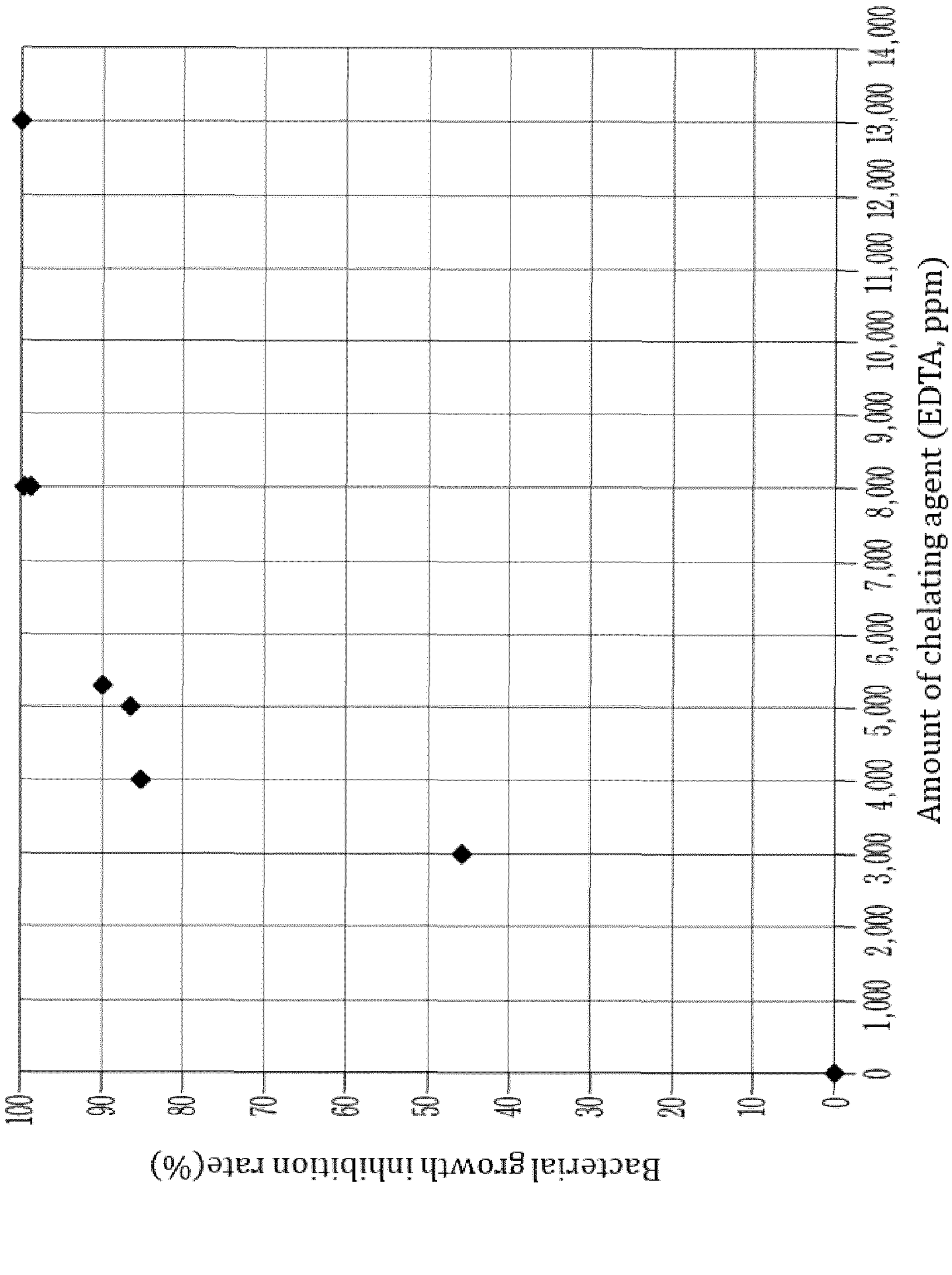
Bacterial growth inhibition rate(%)
0
10
20
30
40
50
60
70
80
90
100
Amount of chelating agent (EDTA, ppm)
0
1,000
2,000
3,000
4,000
5,000
6,000
7,000
8,000
9,000
10,000
11,000
12,000
13,000
14,000

【FIG. 2】

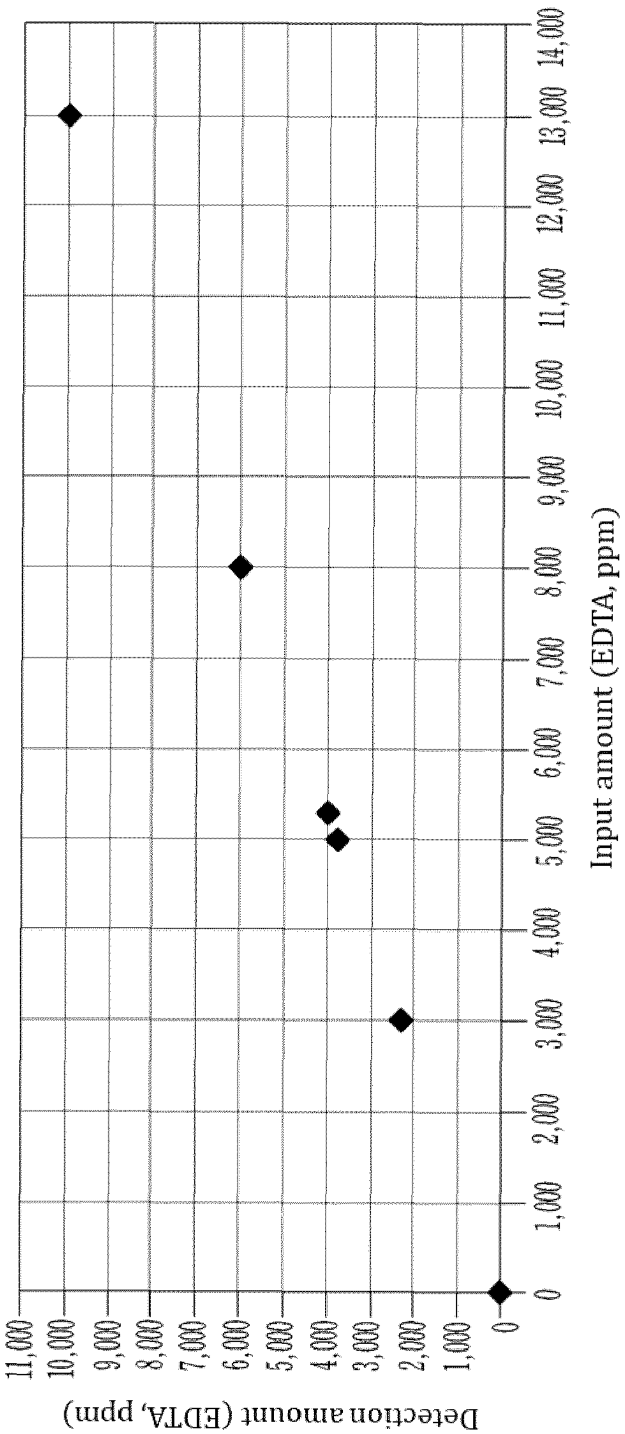
Input amount (EDTA, ppm)
0
1,000
2,000
3,000
4,000
5,000
6,000
7,000
8,000
9,000
10,000
11,000
12,000
13,000
14,000
Detection amount (EDTA, ppm)
0
1,000
2,000
3,000
4,000
5,000
6,000
7,000
8,000
9,000
10,000
11,000

## INTERNATIONAL SEARCH REPORT

International application No.
**PCT/KR2023/021361**

**A. CLASSIFICATION OF SUBJECT MATTER**

**C08K 3/30**(2006.01)i; **C08J 3/24**(2006.01)i; **C08F 2/44**(2006.01)i; **C08K 5/17**(2006.01)i; **C08K 5/092**(2006.01)i; **C08K 5/13**(2006.01)i

According to International Patent Classification (IPC) or to both national classification and IPC

**B. FIELDS SEARCHED**

Minimum documentation searched (classification system followed by classification symbols)

C08K 3/30(2006.01); A61F 13/15(2006.01); A61L 15/60(2006.01); C08F 2/01(2006.01); C08F 2/10(2006.01); C08F 20/04(2006.01); C08J 3/12(2006.01); C08J 3/24(2006.01); C08L 101/12(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 고흡수성 수지(super absorbent polymer), 가교제(crosslinking agent), 박테리아(bacteria), 킬레이트제(chelating agent), 과황산염(persulfate), 쵸핑(chopping)

**C. DOCUMENTS CONSIDERED TO BE RELEVANT**

| Category* | Citation of document, with indication, where appropriate, of the relevant passages | Relevant to claim No. |
|---|---|---|
| Y | KR 10-2018-0087049 A (LG CHEM, LTD.) 01 August 2018 (2018-08-01)<br>See paragraphs [0020]-[0026], [0045]-[0051], [0060], [0074], [0075], [0079], [0081], [0108], [0110], [0146], [0150], [0152], [0154], [0156], [0160], [0168], [0170], [0171] and [0189]. | 1-18 |
| Y | KR 10-2007-0104663 A (NIPPON SHOKUBAI CO., LTD.) 26 October 2007 (2007-10-26)<br>See paragraphs [0022]-[0029], [0098] and [0104]. | 1-18 |
| A | KR 10-2016-0084671 A (HANWHA CHEMICAL CORPORATION) 14 July 2016 (2016-07-14)<br>See claims 1-10. | 1-18 |
| A | JP 2004-346089 A (SUMITOMO SEIKA CHEM. CO., LTD.) 09 December 2004 (2004-12-09)<br>See claims 1-8. | 1-18 |
| A | WO 2009-123193 A1 (NIPPON SHOKUBAI CO., LTD.) 08 October 2009 (2009-10-08)<br>See claims 1-21. | 1-18 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **01 April 2024** | **01 April 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.
**PCT/KR2023/021361**

| Patent document cited in search report | Publication date (day/month/year) | Patent family member(s) | Publication date (day/month/year) |
|---|---|---|---|
| KR 10-2018-0087049 A | 01 August 2018 | CN 108779265 A | 09 November 2018 |
| | | EP 3406655 A1 | 28 November 2018 |
| | | JP 2019-518821 A | 04 July 2019 |
| | | JP 6728396 B2 | 22 July 2020 |
| | | KR 10-2086058 B1 | 06 March 2020 |
| | | US 11712678 B2 | 01 August 2023 |
| | | US 2020-0164344 A1 | 28 May 2020 |
| | | WO 2018-139768 A1 | 02 August 2018 |
| KR 10-2007-0104663 A | 26 October 2007 | AU 2006-216015 A1 | 24 August 2006 |
| | | BR PI0607725 A2 | 06 October 2009 |
| | | CN 101120038 A | 06 February 2008 |
| | | CN 104086938 A | 08 October 2014 |
| | | CN 1847289 A | 18 October 2006 |
| | | DE 602006001215 D1 | 03 July 2008 |
| | | EP 1690887 A1 | 16 August 2006 |
| | | EP 1848758 A1 | 31 October 2007 |
| | | JP 2006-297373 A | 02 November 2006 |
| | | JP 2006-299234 A | 02 November 2006 |
| | | JP 2012-086221 A | 10 May 2012 |
| | | JP 2014-039927 A | 06 March 2014 |
| | | JP 2016-033224 A | 10 March 2016 |
| | | JP 4965865 B2 | 04 July 2012 |
| | | JP 5046522 B2 | 10 October 2012 |
| | | JP 5625003 B2 | 12 November 2014 |
| | | JP 5903086 B2 | 13 April 2016 |
| | | JP 6025953 B2 | 16 November 2016 |
| | | MX 2007009284 A | 29 January 2008 |
| | | RU 2007134385 A | 27 March 2009 |
| | | RU 2364611 C2 | 20 August 2009 |
| | | SG 159540 A1 | 30 March 2010 |
| | | TW 200706566 A | 16 February 2007 |
| | | TW I406883 B | 01 September 2013 |
| | | US 2006-0183828 A1 | 17 August 2006 |
| | | US 2010-0062252 A1 | 11 March 2010 |
| | | US 8182916 B2 | 22 May 2012 |
| | | WO 2006-088115 A1 | 24 August 2006 |
| KR 10-2016-0084671 A | 14 July 2016 | AR 103356 A1 | 03 May 2017 |
| | | TW 201630944 A | 01 September 2016 |
| | | WO 2016-111491 A1 | 14 July 2016 |
| JP 2004-346089 A | 09 December 2004 | BR PI0409259 A | 28 March 2006 |
| | | CN 100422267 C | 01 October 2008 |
| | | CN 1771296 A | 10 May 2006 |
| | | DE 602004029348 D1 | 11 November 2010 |
| | | EP 1616911 A1 | 18 January 2006 |
| | | JP 3979959 B2 | 19 September 2007 |
| | | KR 10-0982839 B1 | 16 September 2010 |
| | | KR 10-2006-0002960 A | 09 January 2006 |
| | | MY 137364 A | 30 January 2009 |
| | | TW 200500406 A | 01 January 2005 |
| | | TW I347963 B | 01 September 2011 |

Form PCT/ISA/210 (patent family annex) (July 2022)

**INTERNATIONAL SEARCH REPORT**
**Information on patent family members**

International application No.
**PCT/KR2023/021361**

| Patent document cited in search report | Publication date (day/month/year) | Patent family member(s) | Publication date (day/month/year) |
|---|---|---|---|
| | | US 2006-0189953 A1 | 24 August 2006 |
| | | US 2011-0068300 A1 | 24 March 2011 |
| | | US 7868075 B2 | 11 January 2011 |
| | | US 8981176 B2 | 17 March 2015 |
| | | WO 2004-090044 A1 | 21 October 2004 |
| WO 2009-123193 A1 | 08 October 2009 | CN 101977939 A | 16 February 2011 |
| | | CN 101977940 A | 16 February 2011 |
| | | EP 2261264 A1 | 15 December 2010 |
| | | EP 2261265 A1 | 15 December 2010 |
| | | JP 5410412 B2 | 05 February 2014 |
| | | JP 5421243 B2 | 19 February 2014 |
| | | US 2011-0021725 A1 | 27 January 2011 |
| | | US 2011-0039961 A1 | 17 February 2011 |
| | | US 8420752 B2 | 16 April 2013 |
| | | US 8912298 B2 | 16 December 2014 |
| | | WO 2009-123197 A1 | 08 October 2009 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020220183171
- KR 1020230188595


### Non-patent literature cited in the description


- **ODIAN**. Principle of Polymerization. Wiley, 1981, 203
- **REINHOLD SCHWALM**. UV Coatings: Basics, Recent Developments and New Application. Elsevier, 2007, 115